# EUROPEAN PATENT SPECIFICATION

(11) **EP 2 870 161 B1**
(45) Date of publication and mention of the grant of the patent: **08.08.2018**
(21) Application number: 13808660.8
(22) Date of filing: 28.06.2013
(51) Int. Cl.: C07D 493/08, A61K 31/341, A61K 31/443, A61K 31/496, A61P 9/10, A61P 41/00, A61P 43/00, A61K 31/34, A61K 31/4525, A61K 31/4178

(54) **OXABICYCLOHEPTANES AND OXABICYCLOHEPTENES FOR THE TREATMENT OF REPERFUSION INJURY**
OXABICYCLOHEPTANE UND OXABICYCLOHEPTENE ZUR BEHANDLUNG VON REPERFUSIONSSCHÄDEN
OXABICYCLOHEPTANES ET OXABICYCLOHEPTÈNES POUR LE TRAITEMENT D'UNE LÉSION DE REPERFUSION

(30) Priority: 29.06.2012 US 201261666535 P; 14.03.2013 US 201361782894 P
(43) Date of publication of application: 13.05.2015
(73) Proprietor: Lixte Biotechnology, Inc., East Setauket, NY 11733 (US)
(72) Inventor: KOVACH, John, S., East Setauket, NY 11733 (US)
(74) Representative: ABG Patentes, S.L.
(86) International application number: PCT/US2013/048697
(87) International publication number: WO 2014/005080

(56) References cited:
- US-A- 4 931 460
- US-A- 5 914 242
- US-A1- 2010 029 683
- US-B2- 8 058 268
- ROBERT M. BELL ET AL: "There is More to Life than Revascularization: Therapeutic Targeting of Myocardial Ischemia/Reperfusion Injury", CARDIOVASCULAR THERAPEUTICS, vol. 29, no. 6, 14 July 2010 (2010-07-14), pages e67-e79, XP055227879, ISSN: 1755-5914, DOI: 10.1111/j.1755-5922.2010.00190.x
- HAUSENLOY ET AL.: 'New directions for protecting the heart against ischaemia-reperfusion injury: targeting the Reperfusion Injury Salvage Kinase (RISK)-pathway.' CARDIOVASC. RES. vol. 61, no. 3, 2004, pages 448 - 460, XP002407967 Retrieved from the Internet: <URL:http://cardiovascres.oxfordjoumals.org /content/61/3/448.full.pdf+html> [retrieved on 2013-11-15]
- ZHOU ET AL.: 'Ascorbate protects against vascular leakage in cecal ligation and puncture-induced septic peritonitis.' AM. J. PHYSIOL. REGUL. INTEGR. COMP. PHYSIOL. vol. 302, no. 4, 2011, pages R409 - R416, XP055184019 Retrieved from the Internet: <URL:http://ajpregu.physiology.org/content/ 302/4/R409.full-text.pdf+html> [retrieved on 2013-11-15]
- SCHÜTTE ET AL.: 'Aerosolized PGE1, PGI2 and nitroprusside protect against vascular leakage in lung ischaemia-reperfusion.' EUR. RESPIR. J. vol. 18, no. 1, 2001, pages 15 - 22, XP055184023 Retrieved from the Internet: <URL:http://erj.ersjournals.com/content/18/ 1/15.full.pdf+html> [retrieved on 2013-11-15]

## Description

### BACKGROUND OF THE INVENTION

Reperfusion is a re-establishment of blood flow and re-oxygentaion of an affected area following an ischemic event and is critical to limit irreversible damage. However, the absence of oxygen and nutrients from the blood creates a condition in which reperfusion injury may occur. The restoration of blood flow after an ischemic event results in inflammation and oxidative damage. Upon restoration of blood flow, white blood cells release inflammatory factors such as interleukins as well as free radicals. The restored blood flow reintroduces oxygen within cells that damages cellular proteins, DNA, and the plasma membrane.

As acute myocardial infarction (MI) remains the leading cause of death worldwide, the possibility that a pharmacologic intervention applied promptly but after the onset of the heart attack would minimize damage to heart tissue caused by reperfusion and therefore be expected to save many lives and reduce the number of individuals with heart failure following excessive cardiac muscle damage after an MI (Yellon and Hausenloy, 2005; Longacre et al, 2011). It has been suggested that a lack of commercial interest in a developing a drug that would likely be used only once in an individual has limited progress in this field (Cohen and Downey, 2011).

At present, the only established intervention that consistently reduces the size of myocardial infarcts in humans is by improving coronary artery flow as soon as possible after an MI either by drugs, which dissolve fresh clots and/or cardiac catheterization with balloon angioplasty with or without placement of a fixed conduit, a stent. These methods of improving coronary artery blood flow (reperfusion) have improved patient care and decreased hospital mortality. However, delay in initiating reperfusion because of travel time to a cardiac center is a serious limitation to applying these treatments to patients with acute cardiac injury. It has also been discovered the reperfusion treatment in and of itself may cause myocardial cell death, a phenomenon called reperfusion injury. Reducing injury caused by reperfusion by pharmacologic means should improve the success of current interventions for acute heart attacks. A drug minimizing tissue damage that could be administered at the time of a MI by emergency personnel prior to arrival at a cardiac center could be a major advance in the care of heart attack victims. Acute injury due to oxygen deprivation leading to myocardial damage is also a significant problem in heart surgery. The incidence of infarction after coronary artery bypass graft surgery has been estimated to be as high as 19% with attendant cardiac morbidity (Longacre et al, 2011) .

Bell et al. 2011 discloses an overview of therapeutic targets for myocardial ischemia and reperfusion injury but does not dislose the PP2A inhibitors of the current invention for use in reducing reperfusion injury in mammalian tissue in a subject or for use in reducing tissue damage in a subject or for use in reducing vascular leakage in a subject due to an acute trauma.

Zhou et al. 2011 discloses that ascorbate protects against vascular leakage in cecal ligation and puncture-induced septic peritonitis but does not dislose the PP2A inhibitors of the current invention for use in reducing reperfusion injury in mammalian tissue in a subject or for use in reducing tissue damage in a subject or for use in reducing vascular leakage in a subject due to an acute trauma.

### SUMMARY OF THE INVENTION

The scope of the present invention is defined in the appended claims. Subject-matter which is not encompassed by the scope of the claims does not form part of the presently claimed invention. The invention is directed to a protein phosphatase 2A (PP2A) inhibitor having the structure: 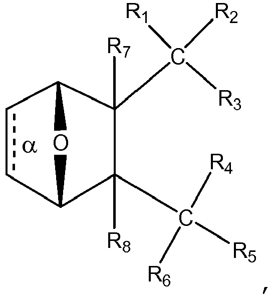 wherein
bond α is present or absent;
R₁ and R₂ is each independently H, O⁻ or OR₉,
where R₉ is H, alkyl, alkenyl, alkynyl or aryl, or R₁ and R₂ together are =O;
R₃ and R₄ are each different, and each is OH, O⁻, OR₉, O(CH₂)₁₋₆R₉, SH, S⁻, SR₉, 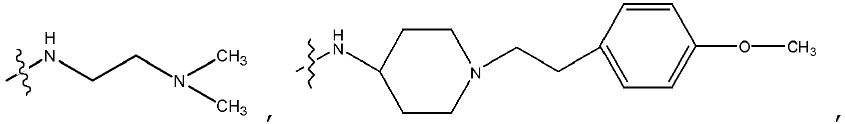 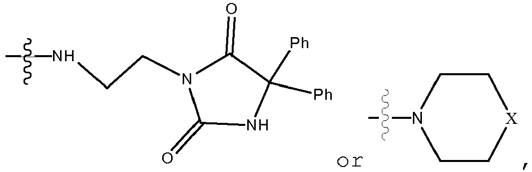 where X is O, S, NR₁₀, or N⁺R₁₀R₁₀,
where each R₁₀ is independently H, alkyl, C₂-C₁₂ alkyl, alkenyl, C₄-C₁₂ alkenyl, alkynyl, aryl, substituted aryl where the substituent is other than chloro when R₁ and R₂ are =O, 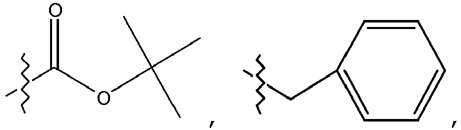 -CH₂CN, -CH₂CO₂R₁₁, -CH₂COR₁₁, - NHR₁₁ or -NH⁺(R₁₁)₂,
wherein each R₁₁ is independently alkyl, alkenyl or alkynyl, or H;
R₅ and R₆ is each independently H, OH, or R₅ and R₆ taken together are =O;
R₇ and R₈ is each independently H, F, Cl, Br, SO₂Ph, CO₂CH₃, or SR₁₂,
where R₁₂ is H, alkyl, alkenyl, alkynyl or aryl; and
each occurrence of alkyl, alkenyl, or alkynyl is branched or unbranched, unsubstituted or substituted,
or a salt, enantiomer or zwitterion of the compound, for use in reducing reperfusion injury in mammalian tissue in a subject or for use in reducing tissue damage due to an acute trauma in a subject or for use in reducing vascular leakage in a subject due to an acute trauma.

### Brief Description of the Figures

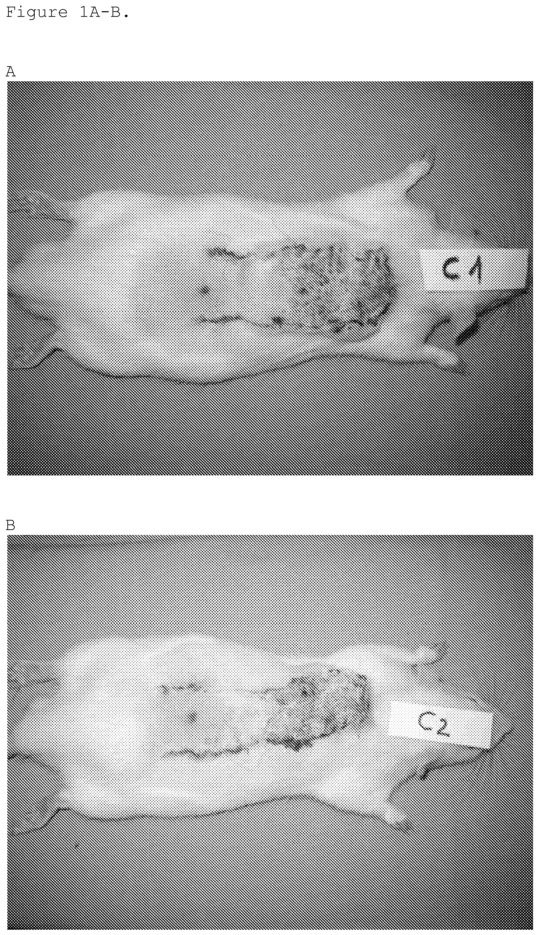

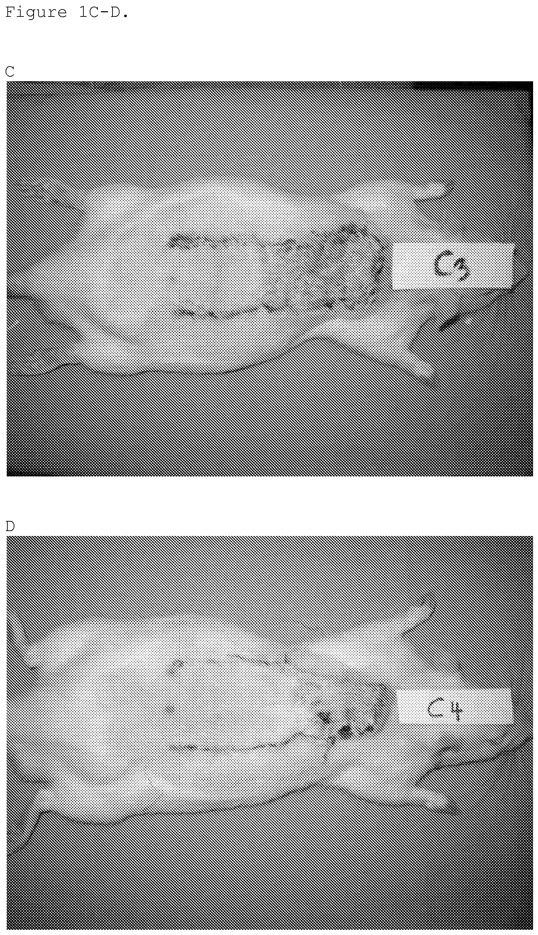

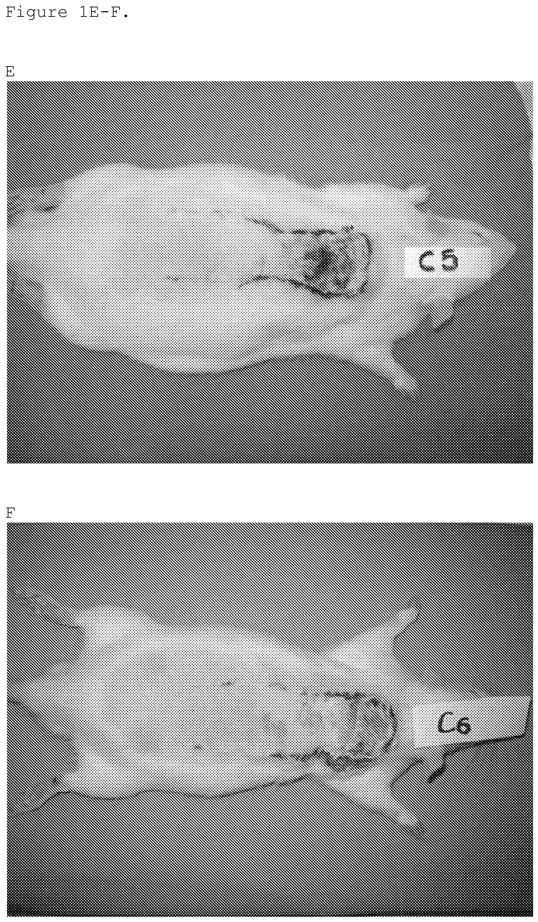

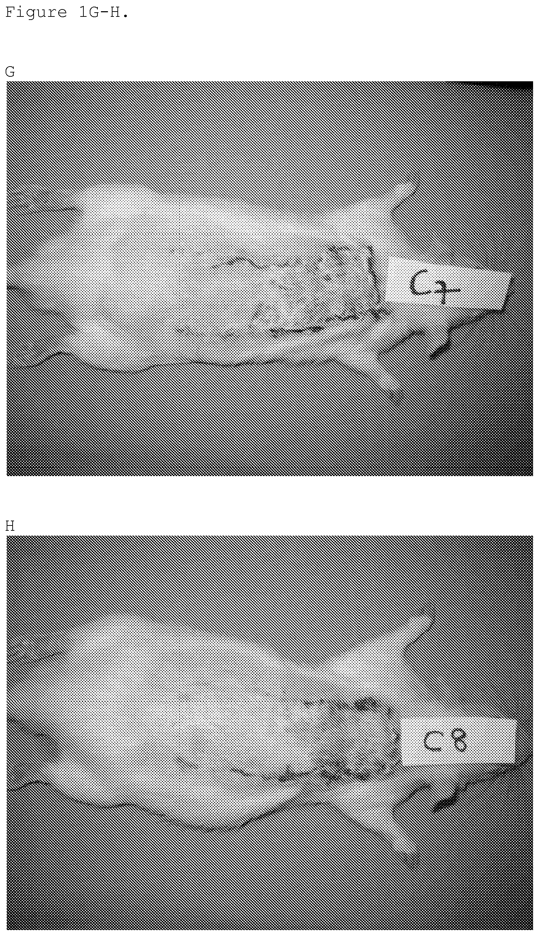

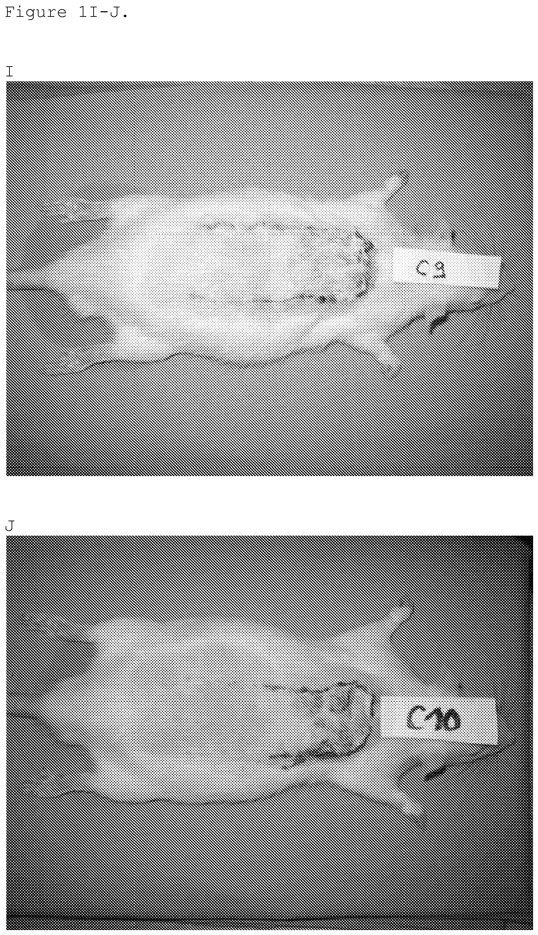

**Figure 1****.** Photographs of in-bred "control" rats 1-10 implanted with a pump containing sodium chloride prior to raising a skin flap. Photographs were taken 7 days after creation of the skin flap. (A) control rat 1; (B) control rat 2; (C) control rat 3; (D) control rat 4; (E) control rat 5; (F) control rat 6; (G) control rat 7; (H) control rat 8; (I) control rat 9; and (J) control rat 10.

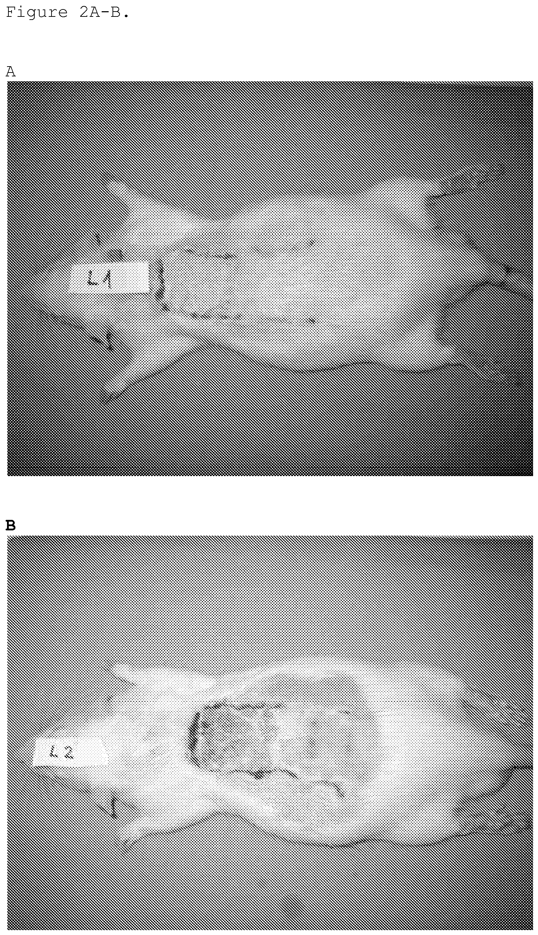

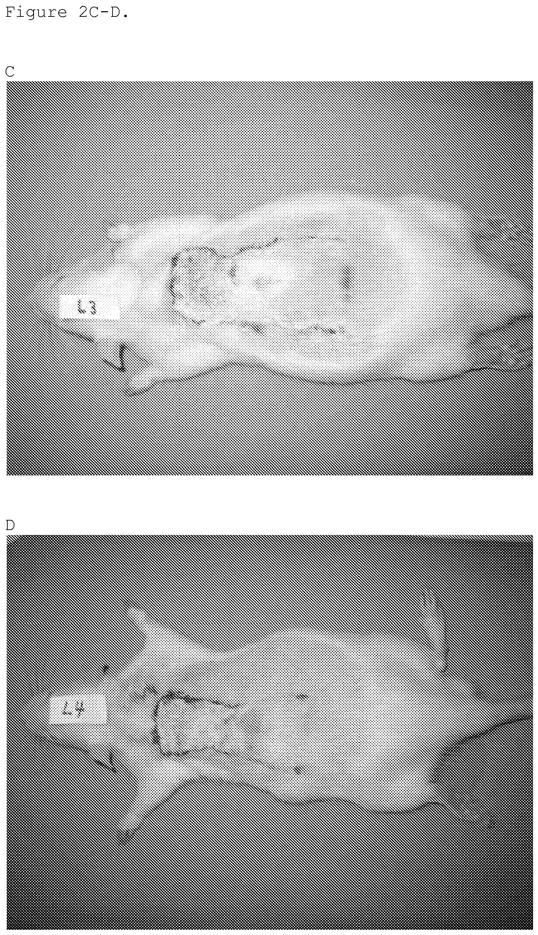

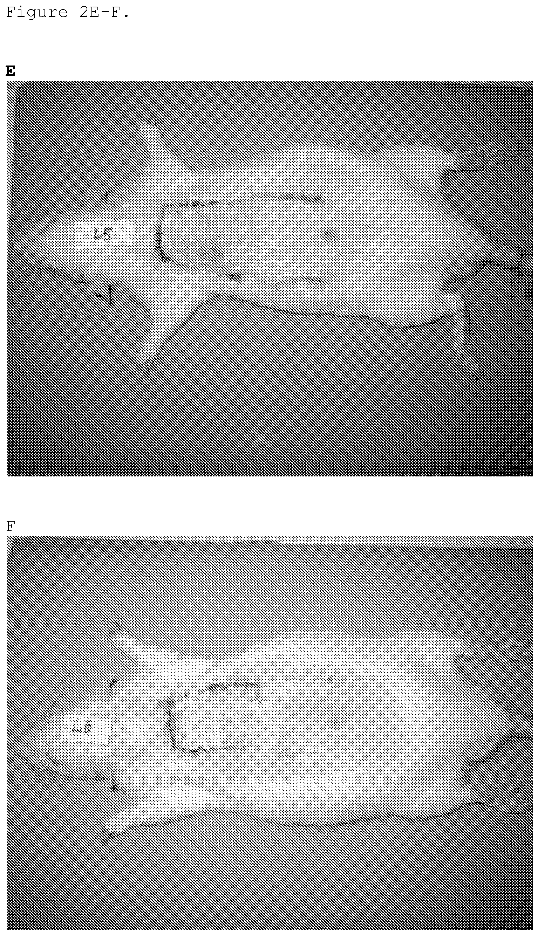

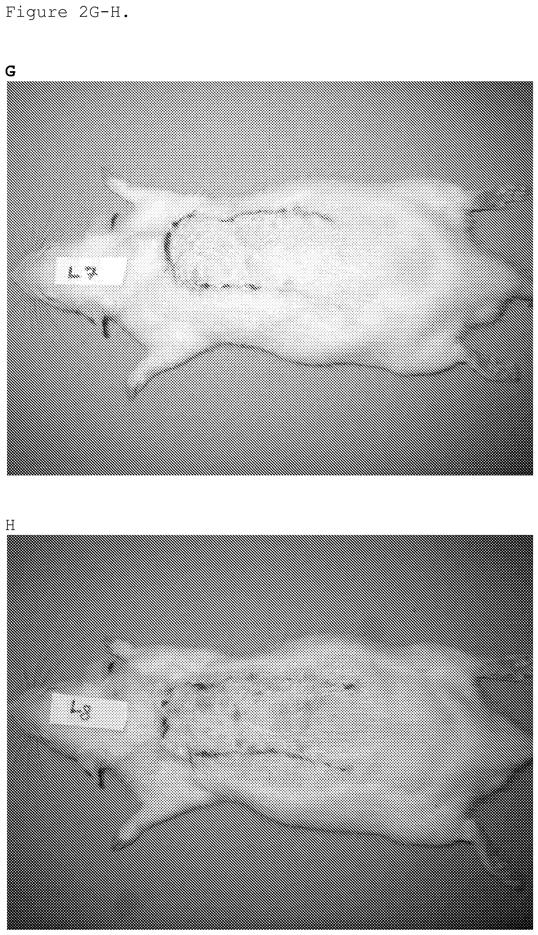

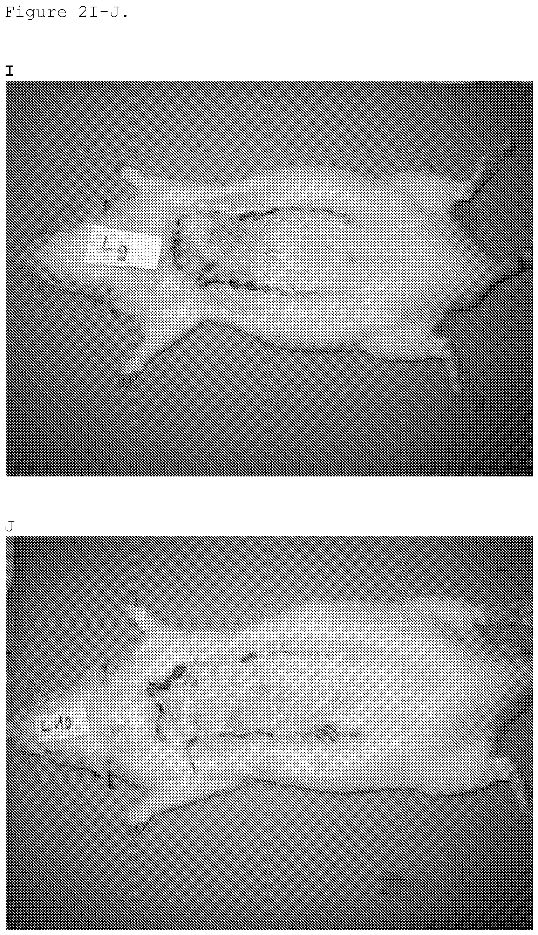

**Figure 2****.** Photographs of in-bred "treatment" rats 1-10 implanted with a pump containing 0.55 mg of LB-100 prior to raising a skin flap. The pump was set to deliver 0.5 µl/hour +/- 10% so as to administer about 12 µl/day for 8 days, four days prior to raising the graft and for the first four days after creation of the graft. Photographs were taken 7 days after creation of the skin flap. (A) treatment rat 1; (B) treatment rat 2; (C) treatment rat 3; (D) treatment rat 4; (E) treatment rat 5; (F) treatment rat 6; (G) treatment rat 7; (H) treatment rat 8; (I) treatment rat 9; and (J) treatment rat 10.

### DETAILED DESCRIPTION OF THE INVENTION

The invention is directed to a protein phosphatase 2A (PP2A) inhibitor having the structure: 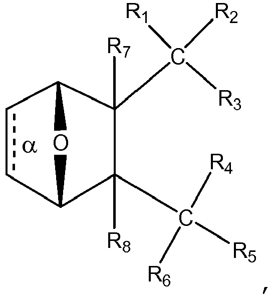 wherein
bond α is present or absent;
R₁ and R₂ is each independently H, O⁻ or OR₉,
where R₉ is H, alkyl, alkenyl, alkynyl or aryl, or R₁ and R₂ together are =O;
R₃ and R₄ are each different, and each is OH, O⁻, OR₉, O(CH2)₁₋₆R₉, SH, S⁻, SR₉, 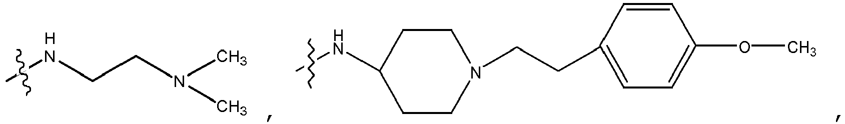 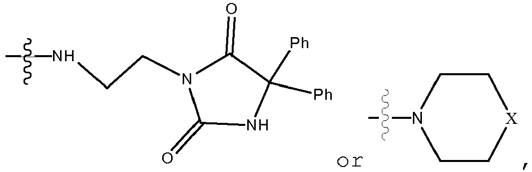 where X is O, S, NR₁₀, or N⁺R₁₀R₁₀,
where each R₁₀ is independently H, alkyl, C₂-C₁₂ alkyl, alkenyl, C₄-C₁₂ alkenyl, alkynyl, aryl, substituted aryl where the substituent is other than chloro when R₁ and R₂ are =O, 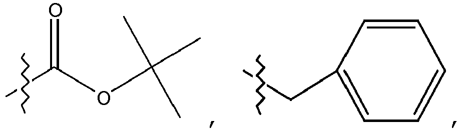 -CH₂CN, -CH₂CO₂R₁₁, -CH₂COR₁₁, - NHR₁₁ or -NH⁺(R₁₁)₂,
wherein each R₁₁ is independently alkyl, alkenyl or alkynyl, or H;
R₅ and R₆ is each independently H, OH, or R₅ and R₆ taken together are =O;
R₇ and R₈ is each independently H, F, Cl, Br, SO₂Ph, CO₂CH₃, or SR₁₂,
where R₁₂ is H, alkyl, alkenyl, alkynyl or aryl; and
each occurrence of alkyl, alkenyl, or alkynyl is branched or unbranched, unsubstituted or substituted,
or a salt, enantiomer or zwitterion of the compound, for use in reducing reperfusion injury in mammalian tissue in a subject or for use in reducing tissue damage due to an acute trauma in a subject or for use in reducing vascular leakage in a subject due to an acute trauma.

In an embodiment, the protein phosphatase 2A (PP2A) inhibitor is for use in reducing reperfusion injury in mammalian tissue in a subject, wherein the tissue is contacted with the protein phosphatase 2A (PP2A) inhibitor.

In some embodiments, the reduction of reperfusion injury comprises increased phosphorylation of Akt in the mammalian tissue that has suffered an ischemia.

In some embodiments, the reduction of reperfusion injury comprises increased activation of Akt in the mammalian tissue that has suffered an ischemia.

In some embodiments, the reduction of reperfusion injury comprises increased phosphorylation of BAD, mdm2, eNOS and/or GSK-3β in the mammalian tissue that has suffered an ischemia.

In some embodiments, the ischemia is caused by a myocardial infarction, stroke or sepsis.

In some embodiments, the tissue is mycocardial tissue, brain tissue or endothelial tissue.

In some embodiments, endothelial dysfunction is reduced.

In an embodiment, the protein phosphatase 2A (PP2A) inhibitor is for use in reducing tissue damage associated with reperfusion injury in the heart of the subject following a myocardial infarction.

In an embodiment, the protein phosphatase 2A (PP2A) inhibitor is for use in reducing vascular leakage associated with reperfusion injury in a subject suffering from sepsis.

In some embodiments, the reperfusion injury is caused by ischemia that is caused by septic shock.

In one embodiment, the protein phosphatase 2A inhibitor has the structure 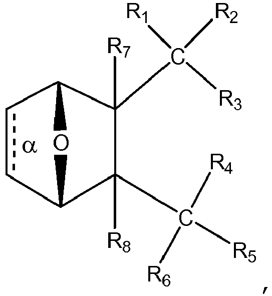 wherein
bond α is present or absent;
R₁ and R₂ is each independently H, O⁻ or OR₉,
where R₈ is H, alkyl, alkenyl, alkynyl or aryl, or R₁ and R₂ together are =O;
R₃ and R₄ are each different, and each is OH, O⁻, OR₉, SH, S⁻, SR₉, 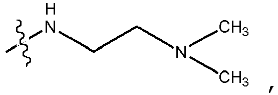 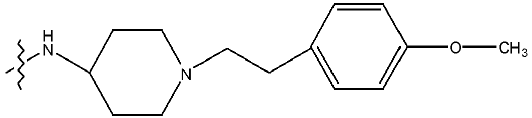 or 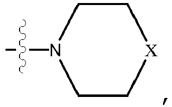 where X is O, S, NR₁₀, or N⁺R₁₀R₁₀,
where each R₁₀ is independently H, alkyl, C₂-C₁₂ alkyl, alkenyl, C₄-C₁₂ alkenyl, alkynyl, aryl, substituted aryl where the substituent is other than chloro when R₁ and R₂ are =O, 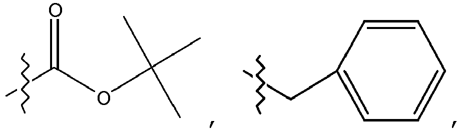 -CH₂CN, -CH₂CO₂R₁₁, -CH₂COR₁₁, - NHR₁₁ or -NH⁺(R₁₁)₂,
where each R₁₁ is independently alkyl, alkenyl, alkynyl, or H;
R₅ and R₆ is each independently H, OH, or R₅ and R₆ taken together are =O; and
R₇ and R₈ is each independently H, F, Cl, Br, SO₂Ph, CO₂CH₃, or SR₁₂, where R₁₂ is H, aryl, alkyl, alkenyl or alkynyl,
or a salt, enantiomer or zwitterion of the compound.

In one embodiment, the protein phosphatase 2A inhibitor has the structure

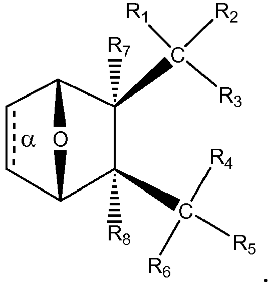

In one embodiment, the protein phosphatase 2A inhibitor has the structure

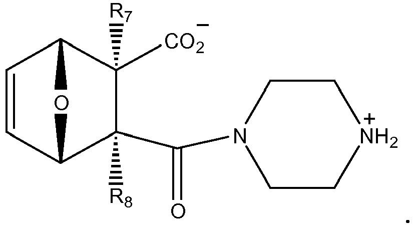

Also disclosed herein is a protein phosphatase 2A inhibitor having the structure

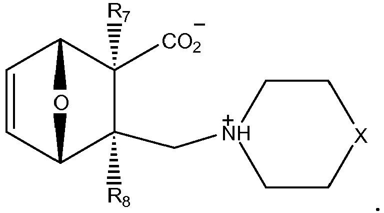

In one embodiment, bond α is present. In another embodiment, bond α is absent.

In one embodiment, R₁ and R₂ together are =O;
R₃ is O⁻ or OR₉, where R₉ is H, methyl, ethyl or phenyl;
R₄ is 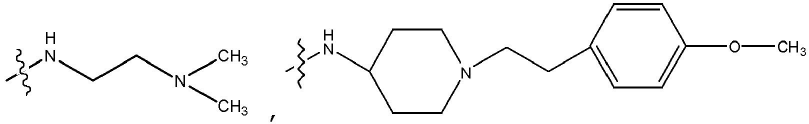 or 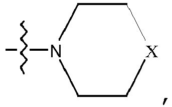 where X is O, S, NR₁₀, or N⁺R₁₀R₁₀,
where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro, 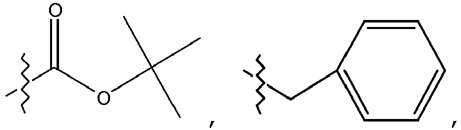 -CH₂CN, -CH₂CO₂R₁₁, -CH₂COR₁₁, - NHR₁₁ or -NH⁺(R₁₁)₂,
where R₁₁ is alkyl, alkenyl or alkynyl, each of which is substituted or unsubstituted, or H;
R₅ and R₆ taken together are =O; and
R₇ and R₈ is each independently H, F, Cl, Br, SO₂Ph, CO₂CH₃, or SR₁₂, where R₁₂ is a substituted or unsubstituted alkyl, alkenyl or alkynyl.

In one embodiment, R₃ is O⁻.

In another embodiment, R₄ is 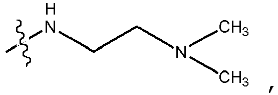 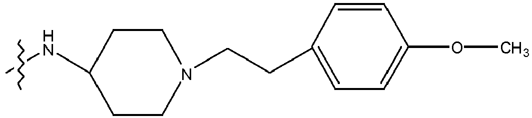 or 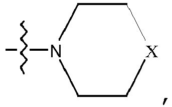 where X is O, NR₁₀, N⁺R₁₀R₁₀
where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro when R₁ and R₂ are =0, 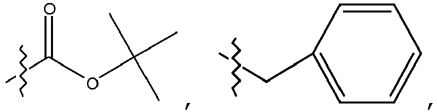 -CH₂CN, -CH₂CO₂R₁₁, -CH₂COR₁₁, - NHR₁₁ or -NH⁺(R₁₁)₂,
where R₁₁ is H or alkyl.

In one embodiment, the protein phosphatase inhibitor 2A has the structure 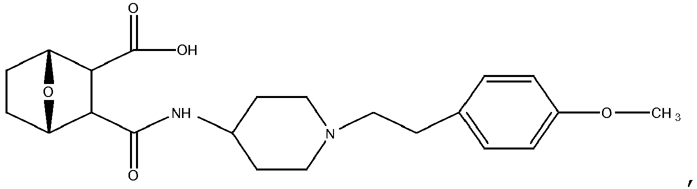 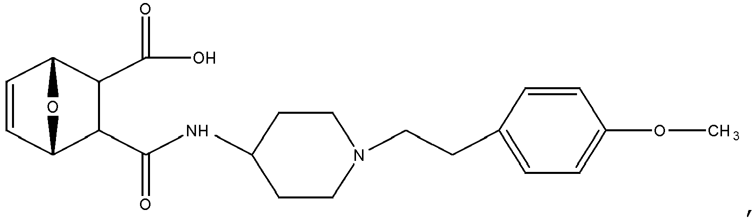 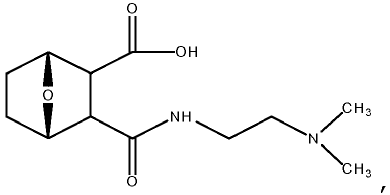 or 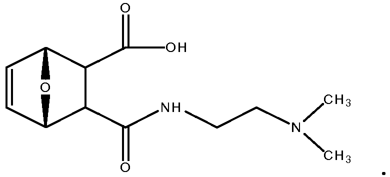 In one embodiment, R₄ is 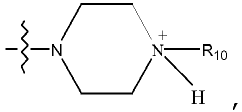 where R₁₀ is H, alkyl, substituted C₂-C₁₂ alkyl, alkenyl, substituted C₄-C₁₂ alkenyl, alkynyl, substituted alkynyl, aryl, substituted aryl where the substituent is other than chloro when R₁ and R₂ are =O, 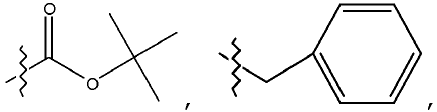 -CH₂CN, -CH₂CO₂R₁₁, -CH₂COR₁₁, -NHR₁₁ or -NH⁺(R₁₁)₂, where R₁₁ is H or alkyl.

In one embodiment, R₄ is

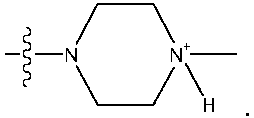

In one embodiment, R₄ is 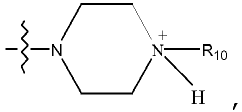 where R₁₀ is

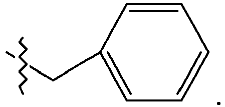

In one embodiment, R₄ is

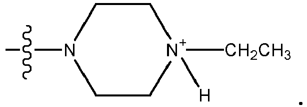

In one embodiment, R₄ is

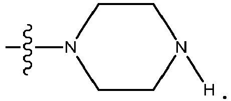

In one embodiment, R₅ and R₆ together are =O. In another embodiment, R₇ and R₈ are each H.

Also disclosed herein is a protein phosphatase 2A (PP2A) inhibitor having the structure: 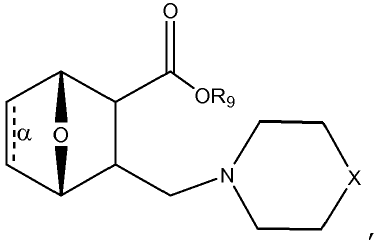 wherein bond α is present or absent;
R₈ is present or absent and when present is H, C₁-C₁₀ alkyl, C₂-C₁₀ alkenyl or phenyl; and X is O, S, NR₁₀ or N⁺R₁₀R₁₀,
where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro, 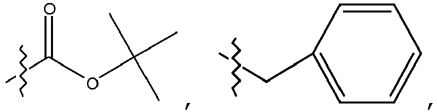 -CH₂CO₂R₁₁, -CH₂COR₁₁, -CH₂CN, or -CH₂CH₂R₁₆, where R₁₁ is H or alkyl, and where R₁₆ is any substitutent that is a precursor to an aziridinyl intermediate,
or a salt, zwitterion or enantiomer of the compound.

Also disclosed herein is a protein phosphatase 2A inhibitor having the structure 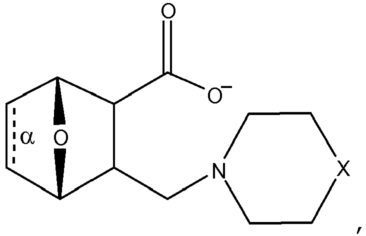 wherein,
bond α is present or absent;
X is O, S, NR₁₀ or N⁺R₁₀R₁₀,
where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro, 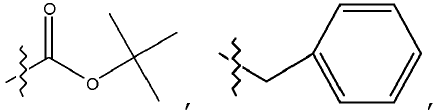 -CH₂CO₂R₁₁, -CH₂COR₁₁, -CH₂CN, or -CH₂CH₂R₁₆, where R₁₁ is H or alkyl, and where R₁₆ is any substitutent that is a aziridinyl intermediate,
or a salt, zwitterion or enantiomer of a compound.

In one embodiment,
X is O or NH⁺R₁₀,
where R₁₀ is H, alkyl, substituted C₂-C₁₂ alkyl, alkenyl, substituted C₄-C₁₂ alkenyl, alkynyl, substituted alkynyl, aryl, substituted aryl where the substituent is other than chloro,

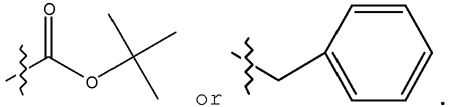

Also disclosed herein is X being -CH₂CH₂R₁₆, where R₁₆ is any substitutent that is a precursor to an aziridinyl intermediate.

In one embodiment, X is O.

In another embodiment, X is NH⁺R₁₀,
where R₁₀ H, alkyl, substituted C₂-C₁₂ alkyl, alkenyl, substituted C₄-C₁₂ alkenyl, alkynyl, substituted alkynyl, aryl, substituted aryl where the substituent is other than chloro,

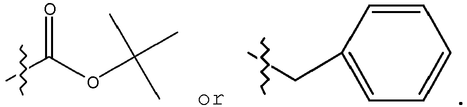

In one embodiment, R₁₀ is methyl. In another embodioment, R₁₀ is

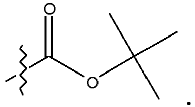

In one embodiment, R₁₀ is

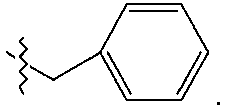

In one embodiment, R₁₀ is ethyl. In another embodiment, R₁₀ is absent.

In one embodiment, the protein phosphatase 2A inhibitor has the structure 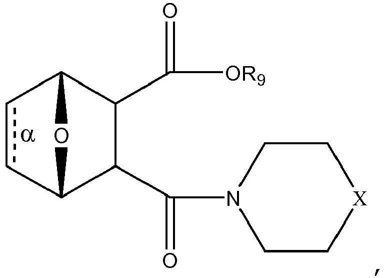 wherein
bond α is present or absent;
R₈ is present or absent and when present is H, alkyl, alkenyl, alkynyl or phenyl; and
X is O, NR₁₀, or N⁺R₁₀R₁₀,
where each R₁₀ is indepdently H, alkyl, substituted C₂-C₁₂ alkyl, alkenyl, substituted C₄-C₁₂ alkenyl, alkynyl, substituted alkynyl, aryl, substituted aryl where the substituent is other than chloro, 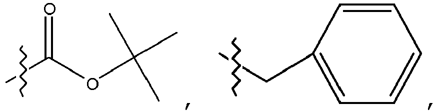 -CH₂CN, -CH₂CO₂R₁₂, or -CH₂COR₁₂,
where R₁₂ is H or alkyl,
or a salt, zwitterion, or enantiomer of the compound.

In one embodiment, the protein phosphatase 2A inhibitor has the structure 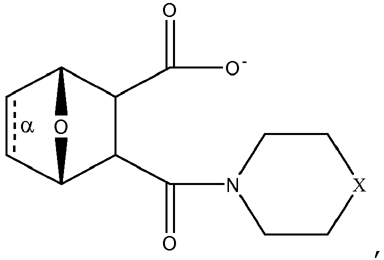 wherein
bond α is present or absent;
X is O or NH⁺R₁₀,
where R₁₀ is H, alkyl, substituted C₂-C₁₂ alkyl, alkenyl, substituted C₄-C₁₂ alkenyl, alkynyl, substituted alkynyl, aryl, substituted aryl where the substituent is other than chloro, 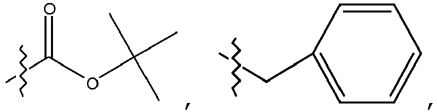 -CH₂CN, -CH₂CO₂R₁₂, or -CH₂COR₁₂, where R₁₂ is H or alkyl.

In one embodiment, bond α is present. In another embodiment, bond α is absent.

In one embodiment, the protein phosphatase 2A inhibitor has the structure 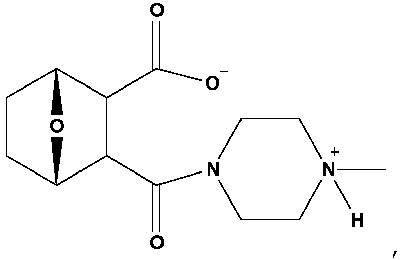 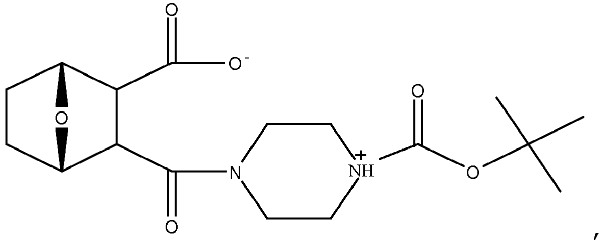 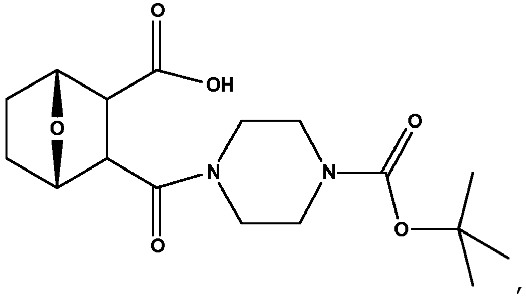 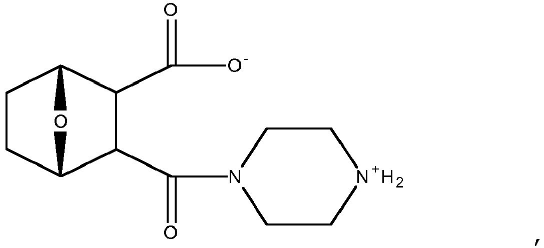 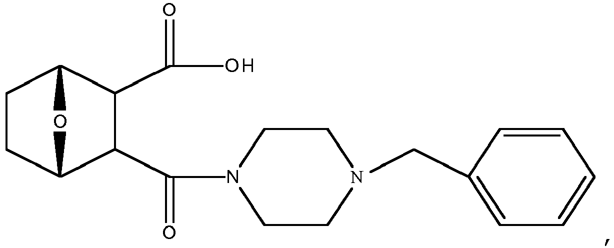 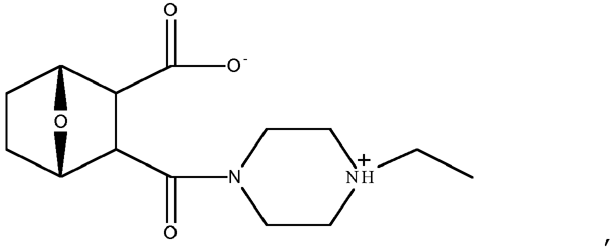 or

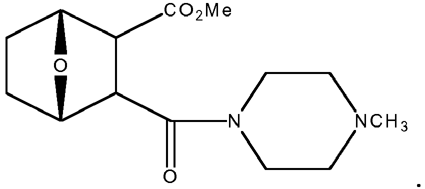

In one embodiment, the protein phosphatase 2A inhibitor has the structure 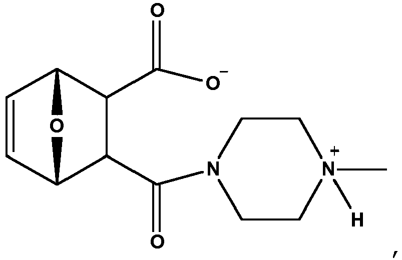 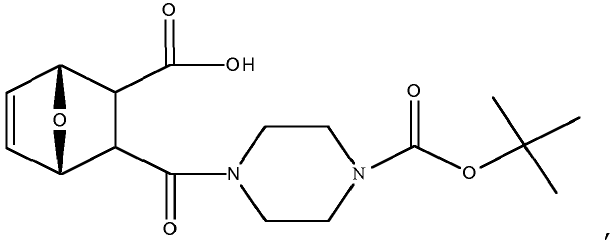 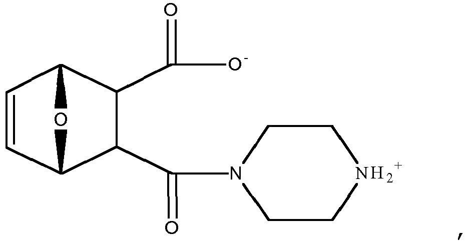 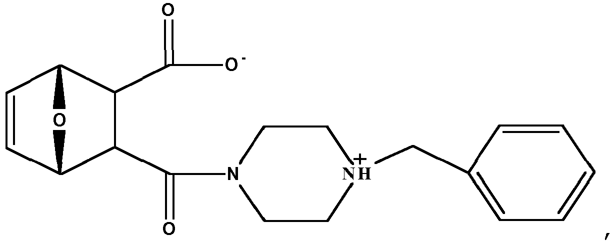 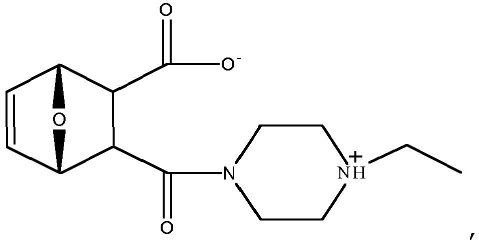 or

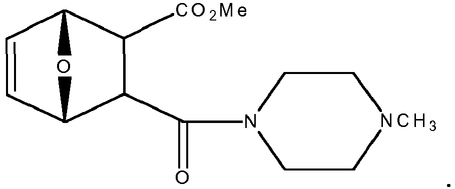

In one embodiment, the protein phosphatase 2A inhibitor has the structure 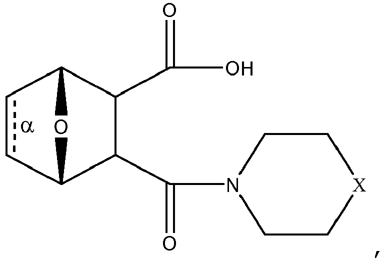 wherein
bond α is present or absent;
X is NH⁺R₁₀,
where R₁₀ is present or absent and when present R₁₀ is is alkyl, substituted C2-C12 alkyl, alkenyl, substituted C4-C12 alkenyl, 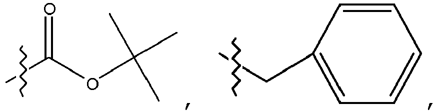 -CH₂CN, -CH₂CO₂R₁₂, or -CH₂COR₁₂, where R₁₂ is H or alkyl.

In one embodiment, the protein phosphatase 2A inhibitor has the structure 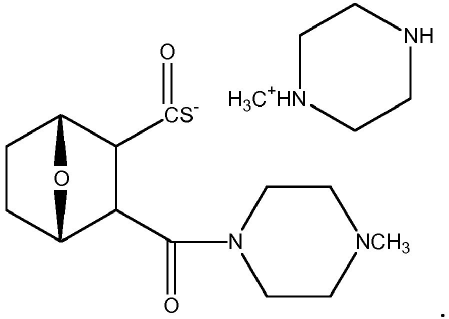 In one embodiment, the protein phosphatase 2A inhibitor has the structure 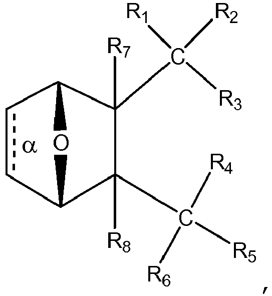 wherein
bond α is present or absent;
R₁ and R₂ is each independently H, O⁻ or OR₉, where R₉ is H, alkyl, substituted alkyl, alkenyl, alkynyl or aryl,
or R₁ and R₂ together are =O;
R₃ and R₄ are each different, and each is O(CH₂)₁₋₆R₉ or OR₉, or 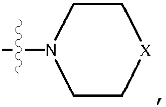 where X is O, S, NR₁₀, or N⁺R₁₀R₁₀,
where each R₁₀ is independently H, alkyl, hydroxyalkyl, C₂-C₁₂ alkyl, alkenyl, C₄-C₁₂ alkenyl, alkynyl, aryl, substituted aryl where the substituent is other than chloro when R₁ and R₂ are =O, 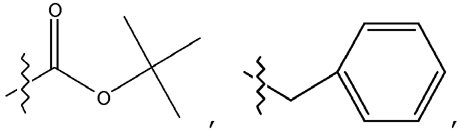 -CH₂CN, -CH₂CO₂R₁₁, -CH₂COR₁₁, -NHR₁₁ or -NH⁺(R₁₁)₂,
where each R₁₁ is independently alkyl, alkenyl or alkynyl, each of which is substituted or unsubstituted, or H;
or R₃ and R₄ are each different and each is OH or 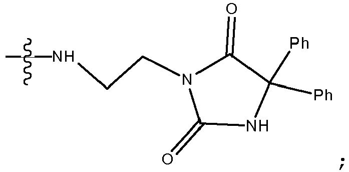 R₅ and R₆ is each independently H, OH, or R₅ and R₆ taken together are =O;
R₇ and R₈ is each independently H, F, Cl, Br, SO₂Ph, CO₂CH₃, or SR₁₂,
where R₁₂ is H, aryl or a substituted or unsubstituted alkyl, alkenyl or alkynyl; and
each occurrence of alkyl, alkenyl, or alkynyl is branched or unbranched, unsubstituted or substituted,
or a salt, enantiomer or zwitterion of the compound.

In one embodiment, the protein phosphatase 2A inhibitor has the structure

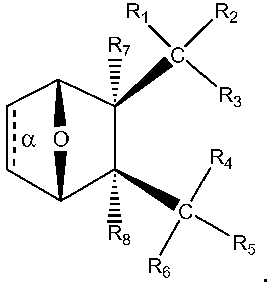

In one embodiment, the bond α is present.

In one embodiment, the bond α is absent.

In one embodiment,
R₃ is OR₉ or O(CH₂)₁₋₆R₉,
where R₉ is aryl, substituted ethyl or substituted phenyl,
wherein the substituent is in the para position of the phenyl; R₄ is 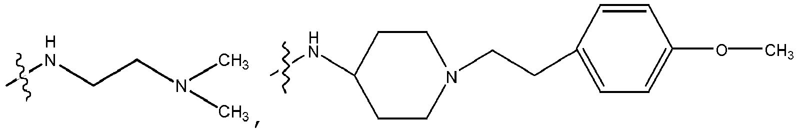 or 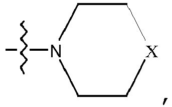 where X is O, S, NR₁₀, or N⁺R₁₀R₁₀,
where each R₁₀ is independently H, alkyl, hydroxyalkyl, substituted C₂-C₁₂ alkyl, alkenyl, substituted C₄-C₁₂ alkenyl, alkynyl, substituted alkynyl, aryl, substituted aryl where the substituent is other than chloro, 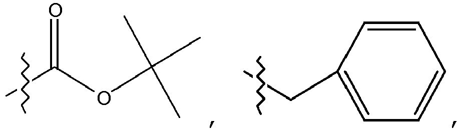 -CH₂CN, -CH₂CO₂R₁₁, -CH₂COR₁₁, -NHR₁₁ or -NH⁺(R₁₁)₂,
where R₁₁ is alkyl, alkenyl or alkynyl, each of which is substituted or unsubstituted, or H;
or where R₃ is OH and R₄ is

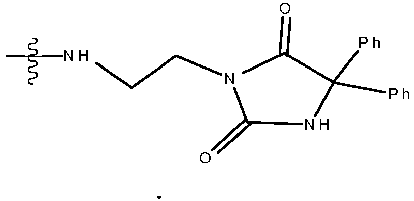

In one embodiment,
R₄ is 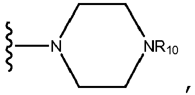 where R₁₀ is alkyl or hydroxylalkyl
or R₄ is 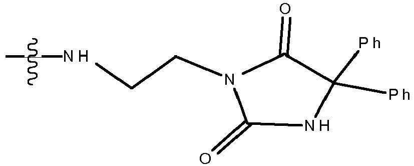 when R₃ is OH.

In one embodiment,
R₁ and R₂ together are =O;
R₃ is OR₉ or O(CH₂)₁₋₂R₉,
where R₉ is aryl, substituted ethyl, or substituted phenyl, wherein the substituent is in the para position of the phenyl;
or R₃ is OH and R₄ is 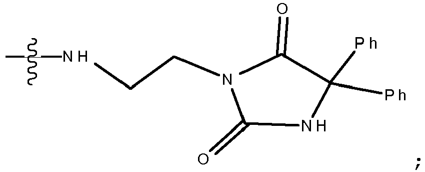 R₄ is 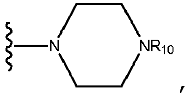 where R₁₀ is alkyl or hydroxyl alkyl;
R₅ and R₆ together are =O; and
R₇ and R₈ are each independently H.

In one embodiment,
R₁ and R₂ together are =O;
R₃ is OH, O(CH₂)R₉, or OR₉,
where R₈ is phenyl or CH₂CCl₃, 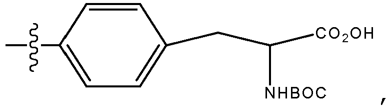 or 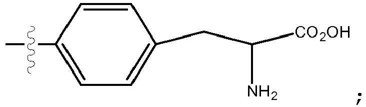 R₄ is 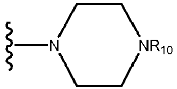 or 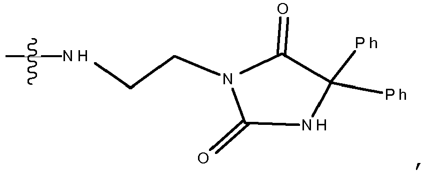 where R₁₀ is CH₃ or CH₃CH₂OH;
R₅ and R₆ together are =O; and
R₇ and R₈ are each independently H.

In one embodiment,
R₃ is OR₉
where R₈ is (CH₂)₁₋₆(CHNHBOC)CO₂H, (CH₂)₁₋₆(CHNH₂)CO₂H, or (CH₂)₁₋₆CCl₃.

In one embodiment,
R₉ is CH₂(CHNHBOC)CO₂H, CH₂(CHNH₂)CO₂H, or CH₂CCl₃.

In one embodiment,
R₃ is O(CH₂)₁₋₆R₉ or O(CH₂)R₉,
where R₉ is phenyl.

In one embodiment,
R₃ is O(CH₂)R₉
where R₉ is phenyl.

In one embodiment,
R₃ is OH and R₄ is

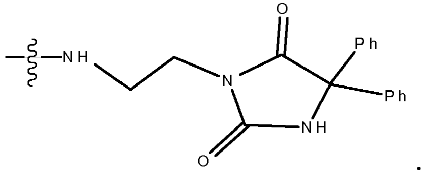

In one embodiment,
R₄ is 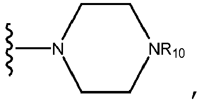 wherein R₁₀ is alkyl or hydroxyalkyl.

In one embodiment, R₁₁ is -CH₂CH₂OH or -CH₃.

In one embodiment, the protein phosphatase 2A inhibitor has the structure 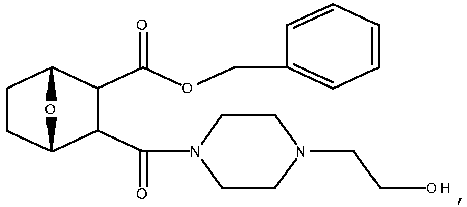 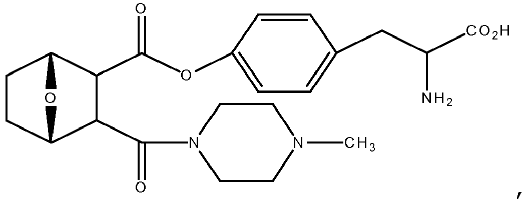 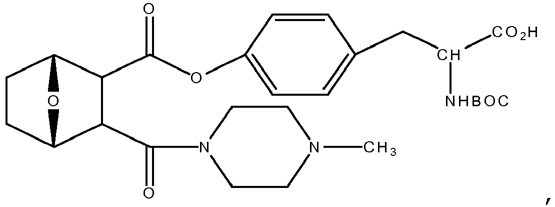 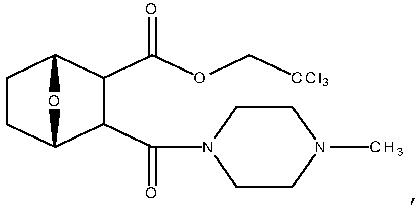 or

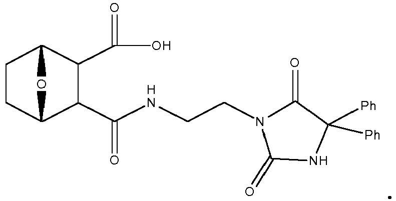

In one embodiment, the protein phosphatase 2A inhibitor has the structure 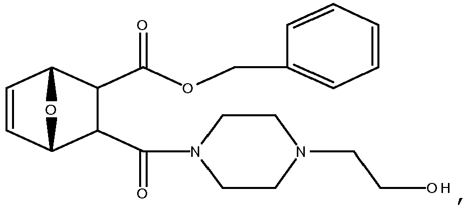 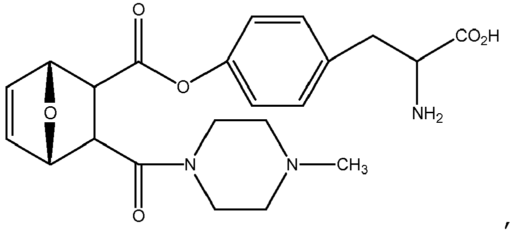 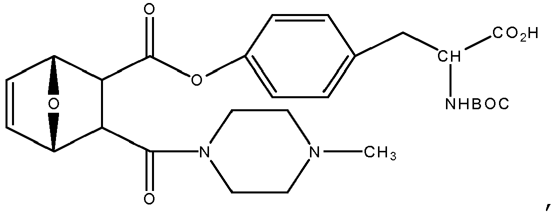 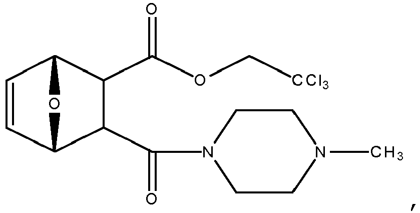 or

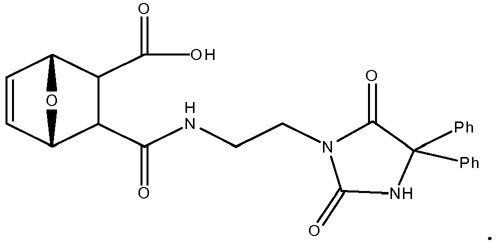

In one embodiment, the protein phosphatase 2A inhibitor is for use in reducing tissue damage associated with reperfusion injury in the heart of the subject following a myocardial infarction; or for use in reducing vascular leakage associated with reperfusion injury in a subject suffering from sepsis.

In one embodiment, the reperfusion injury is tissue damage associated with reperfusion injury in the heart of the subject following myocardial infarction and the tissue is myocardial tissue.

In one embodiment, the reperfusion injury is tissue damage due to an acute trauma in the subject and the tissue is endothelial tissue.

### Definitions

As used herein, and unless otherwise stated, each of the following terms shall have the definition set forth below.

In particular, the invention is directed to the treatment or prevention of reperfusion injury.

As used herein, "reperfusion injury" is tissue damage, tissue death, cell damage, cell death, vascular leakage or endothelial dysfunction caused when blood supply returns to tissue, cells or blood vessels after a period of ischemia or lack of oxygen.

As used herein, "myocardial infarction" (MI), also known as a heart attack, is an infarction of the heart, causing cardiac tissue damage. This is most commonly due to occlusion (blockage) of a coronary artery following the rupture of a vulnerable atherosclerotic plaque, which is an unstable collection of lipids (fatty acids) and white blood cells (especially macrophages) in the wall of an artery. The resulting ischemia (restriction in blood supply) and oxygen shortage, if left untreated for a sufficient period of time, can cause damage or death of heart muscle tissue (myocardium) due to reperfusion injury.

Examples of conditions caused by ischemia and that result in reperfusion injury include myocardial infarction; cerebral infarction (stroke) due to a disturbance in the blood vessels supplying blood to the brain; pulmonary infarction or lung infarction; Splenic infarction occurs when the splenic artery or one of its branches are occluded, for example by a blood clot; Limb infarction caused by arterial embolisms; skeletal muscle infarction caused by diabetes mellitus; bone infarction; testicle infarction; and sepsis.

As used herein, a "symptom" associated with reperfusion injury includes any clinical or laboratory manifestation associated with reperfusion injury and is not limited to what the subject can feel or observe.

As used herein, "treatment of the diseases" or "treating", e.g. of reperfusion injury, encompasses inducing prevention, inhibition, regression, or stasis of the disease or a symptom or condition associated with the disease.

As used herein, "inhibition" of disease progression or disease complication in a subject means preventing or reducing the disease progression and/or disease complication in the subject.

As used herein, "alkyl" includes both branched and straight-chain saturated aliphatic hydrocarbon groups having the specified number of carbon atoms. Thus, C₁-Cₙ as in "C₁-Cₙ alkyl" is defined to include groups having 1, 2, ...., n-1 or n carbons in a linear or branched arrangement, and specifically includes methyl, ethyl, propyl, butyl, pentyl, hexyl, and so on. An embodiment can be C₁-C₁₂ alkyl. "Alkoxy" represents an alkyl group as described above attached through an oxygen bridge.

The term "alkenyl" refers to a non-aromatic hydrocarbon radical, straight or branched, containing at least 1 carbon to carbon double bond, and up to the maximum possible number of non-aromatic carbon-carbon double bonds may be present. Thus, C₂-Cₙ alkenyl is defined to include groups having 2...., n-1 or n carbons. For example, "C₂-C₆ alkenyl" means an alkenyl radical having 2, 3, 4, 5, or 6 carbon atoms, and at least 1 carbon-carbon double bond, and up to, for example, 3 carbon-carbon double bonds in the case of a C₆ alkenyl, respectively. Alkenyl groups include ethenyl, propenyl, butenyl and cyclohexenyl. As described above with respect to alkyl, the straight, branched or cyclic portion of the alkenyl group may contain double bonds and may be substituted if a substituted alkenyl group is indicated. An embodiment can be C₂-C₁₂ alkenyl.

The term "alkynyl" refers to a hydrocarbon radical straight or branched, containing at least 1 carbon to carbon triple bond, and up to the maximum possible number of non-aromatic carbon-carbon triple bonds may be present. Thus, C₂-Cₙ alkynyl is defined to include groups having 2...., n-1 or n carbons. For example, "C₂-C₆ alkynyl" means an alkynyl radical having 2 or 3 carbon atoms, and 1 carbon-carbon triple bond, or having 4 or 5 carbon atoms, and up to 2 carbon-carbon triple bonds, or having 6 carbon atoms, and up to 3 carbon-carbon triple bonds. Alkynyl groups include ethynyl, propynyl and butynyl. As described above with respect to alkyl, the straight or branched portion of the alkynyl group may contain triple bonds and may be substituted if a substituted alkynyl group is indicated. An embodiment can be a C₂-Cₙ alkynyl.

As used herein, "aryl" means any stable monocyclic or bicyclic carbon ring of up to 10 atoms in each ring, wherein at least one ring is aromatic. Examples of such aryl elements include phenyl, naphthyl, tetrahydro-naphthyl, indanyl, biphenyl, phenanthryl, anthryl or acenaphthyl. In cases where the aryl substituent is bicyclic and one ring is non-aromatic, it is understood that attachment is via the aromatic ring. The substituted aryls included in this invention include substitution at any suitable position with amines, substituted amines, alkylamines, hydroxys and alkylhydroxys, wherein the "alkyl" portion of the alkylamines and alkylhydroxys is a C₂-Cₙ alkyl as defined hereinabove. The substituted amines may be substituted with alkyl, alkenyl, alkynl, or aryl groups as hereinabove defined.

The alkyl, alkenyl, alkynyl, and aryl substituents may be substituted or unsubstituted, unless specifically defined otherwise. For example, a (C₁-C₆) alkyl may be substituted with one or more substituents selected from OH, oxo, halogen, alkoxy, dialkylamino, or heterocyclyl, such as morpholinyl, piperidinyl, and so on.

In the compounds of the present invention, alkyl, alkenyl, and alkynyl groups can be further substituted by replacing one or more hydrogen atoms by non-hydrogen groups described herein to the extent possible. These include halo, hydroxy, mercapto, amino, carboxy, cyano and carbamoyl.

The term "substituted" as used herein means that a given structure has a substituent which can be an alkyl, alkenyl, or aryl group as defined above. The term shall be deemed to include multiple degrees of substitution by a named substitutent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results.

As used herein, a "compound" is a small molecule that does not include proteins, peptides or amino acids.

As used herein, an "isolated" compound is a compound isolated from a crude reaction mixture or from a natural source following an affirmative act of isolation. The act of isolation necessarily involves separating the compound from the other components of the mixture or natural source, with some impurities, unknown side products and residual amounts of the other components permitted to remain. Purification is an example of an affirmative act of isolation.

As used herein, "administering" an agent may be performed using any of the various methods or delivery systems well known to those skilled in the art. The administering can be performed, for example, orally, parenterally, intraperitoneally, intravenously, intraarterially, transdermally, sublingually, intramuscularly, rectally, transbuccally, intranasally, liposomally, via inhalation, vaginally, intraoccularly, via local delivery, subcutaneously, intraadiposally, intraarticularly, intrathecally, into a cerebral ventricle, intraventicularly, intratumorally, into cerebral parenchyma or intraparenchchymally.

The following delivery systems, which employ a number of routinely used pharmaceutical carriers, may be used but are only representative of the many possible systems envisioned for administering compositions in accordance with the invention.

Injectable drug delivery systems include solutions, suspensions, gels, microspheres and polymeric injectables, and can comprise excipients such as solubility-altering agents (e.g., ethanol, propylene glycol and sucrose) and polymers (e.g., polycaprylactones and PLGA's).

Other injectable drug delivery systems include solutions, suspensions, gels. Oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc).

Implantable systems include rods and discs, and can contain excipients such as PLGA and polycaprylactone.

Oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc).

Transmucosal delivery systems include patches, tablets, suppositories, pessaries, gels and creams, and can contain excipients such as solubilizers and enhancers (e.g., propylene glycol, bile salts and amino acids), and other vehicles (e.g., polyethylene glycol, fatty acid esters and derivatives, and hydrophilic polymers such as hydroxypropylmethylcellulose and hyaluronic acid).

Dermal delivery systems include, for example, aqueous and nonaqueous gels, creams, multiple emulsions, microemulsions, liposomes, ointments, aqueous and nonaqueous solutions, lotions, aerosols, hydrocarbon bases and powders, and can contain excipients such as solubilizers, permeation enhancers (e.g., fatty acids, fatty acid esters, fatty alcohols and amino acids), and hydrophilic polymers (e.g., polycarbophil and polyvinylpyrolidone) . In one embodiment, the pharmaceutically acceptable carrier is a liposome or a transdermal enhancer.

Solutions, suspensions and powders for reconstitutable delivery systems include vehicles such as suspending agents (e.g., gums, xanthans, cellulosics and sugars), humectants (e.g., sorbitol), solubilizers (e.g., ethanol, water, PEG and propylene glycol), surfactants (e.g., sodium lauryl sulfate, Spans, Tweens, and cetyl pyridine), preservatives and antioxidants (e.g., parabens, vitamins E and C, and ascorbic acid), anti-caking agents, coating agents, and chelating agents (e.g., EDTA).

As used herein, "pharmaceutically acceptable carrier" refers to a carrier or excipient that is suitable for use with humans and/or animals without undue adverse side effects (such as toxicity, irritation, and allergic response) commensurate with a reasonable benefit/risk ratio. It can be a pharmaceutically acceptable solvent, suspending agent or vehicle, for delivering the instant compounds to the subject.

The compounds used in the present invention may be in a salt form. As used herein, a "salt" is a salt of the instant compounds which has been modified by making acid or base salts of the compounds. In the case of compounds used to treat an infection or disease, the salt is pharmaceutically acceptable. Examples of pharmaceutically acceptable salts include mineral or organic acid salts of basic residues such as amines; alkali or organic salts of acidic residues such as phenols. The salts can be made using an organic or inorganic acid. Such acid salts are chlorides, bromides, sulfates, nitrates, phosphates, sulfonates, formates, tartrates, maleates, malates, citrates, benzoates, salicylates, ascorbates, and the like. Phenolate salts are the alkaline earth metal salts, sodium, potassium or lithium. The term "pharmaceutically acceptable salt" in this respect, refers to the relatively non-toxic, inorganic and organic acid or base addition salts of compounds of the present invention. These salts can be prepared in situ during the final isolation and purification of the compounds of the invention, or by separately reacting a purified compound of the invention in its free base or free acid form with a suitable organic or inorganic acid or base, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, e.g., Berge et al. (1977) "Pharmaceutical Salts", J. Pharm. Sci. 66:1-19).

As used herein, an "amount" or "dose" of an agent measured in milligrams refers to the milligrams of agent present in a drug product, regardless of the form of the drug product.

As used herein, the term "therapeutically effective amount" or "effective amount" refers to the quantity of a component that is sufficient to yield a desired therapeutic response without undue adverse side effects (such as toxicity, irritation, or allergic response) commensurate with a reasonable benefit/risk ratio when used in the manner of this invention. The specific effective amount will vary with such factors as the particular condition being treated, the physical condition of the patient, the type of mammal being treated, the duration of the treatment, the nature of concurrent therapy (if any), and the specific formulations employed and the structure of the compounds or its derivatives.

Where a range is given in the specification it is understood that the range includes all integers and 0.1 units within that range, and any sub-range thereof. For example, a range of 77 to 90% is a disclosure of 77, 78, 79, 80, and 81% etc.

As used herein, "about" with regard to a stated number encompasses a range of +one percent to -one percent of the stated value. By way of example, about 100 mg/kg therefore includes 99, 99.1, 99.2, 99.3, 99.4, 99.5, 99.6, 99.7, 99.8, 99.9, 100, 100.1, 100.2, 100.3, 100.4, 100.5, 100.6, 100.7, 100.8, 100.9 and 101 mg/kg. Accordingly, about 100 mg/kg includes, in an embodiment, 100 mg/kg. It is understood that where a parameter range is provided, all integers within that range, and tenths thereof, are also provided by the invention. For example, "0.2-5 mg/kg/day" is a disclosure of 0.2 mg/kg/day, 0.3 mg/kg/day, 0.4 mg/kg/day, 0.5 mg/kg/day, 0.6 mg/kg/day etc. up to 5.0 mg/kg/day.

All combinations of the various elements described herein are within the scope of the invention.

This invention will be better understood by reference to the Experimental Details which follow.

### Experimental Details

### Example 1. Synthesis of LB-107

LB-107 (**5)** was prepared by reacting acid **3** with N-methylpiperizine (**4**) in the presence of EDC. In order to prepare **5** in better yields three different methods were attempted. In the first method, one pot reaction on LB-100 using thionyl chloride in methanol was attempted but no product was observed. In the second method, acid chloride of LB-100 was allowed to react with methanol in presence of triethylamine/DMAP to give the desired methyl ester. The methyl ester thus obtained was in low yields and the separation of triethylamine from the product was also tedious. Hence a two-step procedure was used. In this third method, endothal (**1**) when heated under reflux in methanol gave the desired monomethylester **3** in 95% yields. Compound **3** when treated with N-methylpiperazine (**4**) in presence of EDC and a catalytic amount of N-hydroxybenzotriazole gave the required methyl ester **5** in 39% yields after purification with column chromatography.

### 7-Oxa-bicyclo [2,2,1]heptane-2,3-dicarboxylic acid monomethyl ester (3):

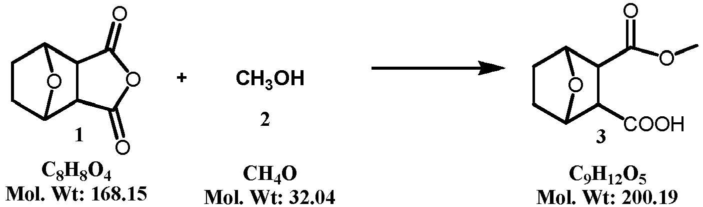

The mixture of exo-7-Oxabicyclo[2.2.1]heptane-2,3-dicarboxylic anhydride (**1**, 10.0 g, 59.5 mmole) and dry methanol (**2**, 50 mL) was heated at reflux temperature for 3.5 h. The reaction mixture became homogeneous during the reflux. The reaction mixture was then cooled down to room temperature and concentrated to give **3** (11.3 g, 95%) as crystalline white material. The crude ¹H NMR was clean enough with no extra peaks. Hence this material was utilized in the next step without further purification. ¹H NMR (DMSO-d₆) δ 1.52 (m, 4H), 2.98 (s, 2H), 3.49 (s, 3H), 4.66 (d, 2H), 12.17 (s, 1H).

### 3-(4-Methylpiperazine-1-carbonyl)-7-oxa-bicyclo[2,2,1]heptane-2-carboxylic acid methyl ester (5):

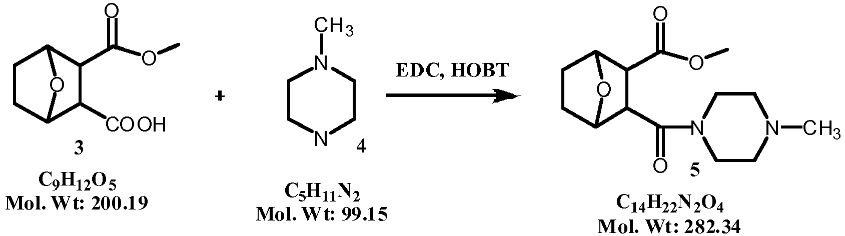

To a mixture of acid **3** (2.00 g, 10.0 mmole) in 50 mL of methylene chloride containing N-hydroxybenzotriazole (98.0 mg, 0.725 mmol) and EDC (2.09 g, 13.5 mmole) was added N-methylpiperazine (**4,** 1.45 g, 14.5 mmole) and the reaction mixture was stirred at room temperature overnight. The reaction mixture was evaporated to dryness and the product purified by column using 5% methanol in methylene chloride to give the required ester **5** as a semi solid (1.89 g, 67%). This was further purified by triturating with isopropyl ether followed by re-crystallization with a mixture of ethyl acetate /Hexane to give a white crystalline material of **5** (LB-107) (1.10 g, yield: 39%, mp 108-109°C). The mother liquor was concentrated and saved for future recrystallization. ¹H NMR (CDCl₃) δ 1.50 (m, 2H), 1.83 (m, 2H), 2.30-2.44 (m, 7H), 2.94 (d, *J*= 9.6 Hz, 1H), 3.10 (d, *J*= 9. 6 Hz, 1H), 3.50 (m, 3H), 3. 71 (m, 4H), 4. 90 (m, 2H), ESMS: 282.

### Example 2. Protein Phosphatase 2A Inhibitors

The compounds used in the method of the present invention are protein phosphatase 2A (PP2A) inhibitors (Lu et al., 2009; US 7,998,957 B2). Compounds LB-100 and LB-102 are inhibitors of PP2A *in vitro* in human cancer cells and in xenografts of human tumor cells in mice when given parenterally in mice. These compounds inhibit the growth of cancer cells in mouse model systems. It has also been shown that another structural homolog of these compounds, LB-107, is active when given orally to mice.

LB100, LB102 or LB107 are tested in an animal model of cardiac ischemia-reperfusion injury.

The structure of LB100 is:

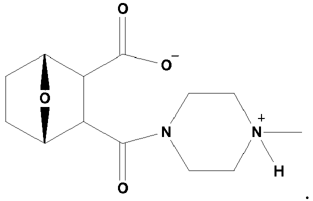

The structure of LB102 is:

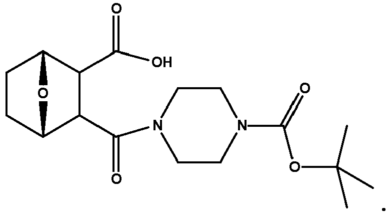

The structure of LB107 is:

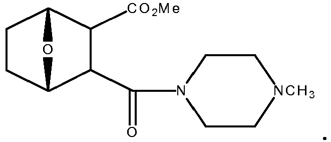

### Example 3. Increase Phosphorylation and Activation of Akt

Compounds LB-100, LB-102, LB-107, and other homlogs of LB-100 disclosed herein increase phosphorylation of Akt in mammalian cells, including cardiac cells, brain cells and endothelial cells. Comopounds LB-100, LB-102 and LB-107 and other homologs of LB-100 disclosed herein reduce dephosphorylation and inactivation of of Akt by protein phosphtase 2A (PP2A) in mammalian cells, including cardiac cells, brain cells and endothelial cells. Comopounds LB-100, LB-102 and LB-107 and other homologs of LB-100 disclosed herein increase activation of Akt by protein phosphtase 2A (PP2A) in mammalian cells, including cardiac cells, brain cells and endothelial cells

### Example 4. Reperfusion Injury

Compounds LB-100, LB-102, LB-107 and other homologs of LB-100 disclosed herein reduce reperfusion injury in mammalian tissue that has suffered from an ischemia. The mammalian tissue includes, but is not limted to, cardiac tissue, brain tissue and endothelial tissue.

### Example 5. Myocardial Infarction

Compounds LB-100, LB-102, LB-107 and other homlogs of LB-100 disclosed herein reduce tissue damage associated with reperfusion injury in the heart of a subject following a myocardial infarction. Compounds LB-100, LB-102, LB-107 and other homlogs of LB-100 disclosed herein prevent tissue damage associated with reperfusion injury in the heart of a subject following a myocardial infarction.

### Example 5. Sepsis

Compounds LB-100, LB-102, LB-107 and other homlogs of LB-100 disclosed herein reduce vascular leakage associated with reperfusion injury in a subject suffering from sepsis. Compounds LB-100, LB-102, LB-107 and other homlogs of LB-100 disclosed herein reduce endothelial dysfunction associated with reperfusion injury in a subject suffering from sepsis.

### Example 6. Study of LB-100 to improve vascular integrity and reduce tissue damage following acute trauma to tissue

The ability of LB-100 to improve vascular integrity and to reduce tissue damage following acute trauma to tissue was tested by analyzing the survival of tissue at the distal end of a flap graft in in-bred rats infused with LB-100 via an implanted pump prior to raising a skin flap.

An ALZET pump (model 1007D, reservoir volume 100ul) was implanted subcutaneously under anesthesia subcutaneously four days before creating a skin flap again under anesthesia. The pump in control animals contained sodium chloride and the pump in treated animals contained 0.55 mg of LB-100. The pump delivers 0.5 ul/hour +/- 10% so each animal received about 12 ul/day for 8 days, 4 days prior to raising the graft and for the first 4 days after creation of the graft. There were 10 animals in each group.

The animals were sacrificed on day 7 following creation of the flap, the amount of necrosis at the end of the flap was measured by planimetry. The area of necrosis at the end of the flap was divided by total surface area of the entire flap and expressed as a percentage. Table 1 shows the area (%) of necrosis in control rats 1-10 vs rats receiving LB-100 (treatment rats).

**Table 1. Area of necrosis in control rats vs rats receiving LB-100.**

| Control | Total | Necrosis | % | Treatment | Total | Necrosis | % |
|---|---|---|---|---|---|---|---|
| 1 | 245751 | 139686 | 56.8404605 | 1 | 180926 | 55473 | 30.6606016 |
| 2 | 222271 | 94119 | 42.3442554 | 2 | 188880 | 63777 | 33.7658831 |
| 3 | 273475 | 142373 | 52.0607002 | 3 | 254862 | 66144 | 25.9528686 |
| 4 | 293026 | 87832 | 29.974132 | 4 | 154749 | 37181 | 24.0266496 |
| 5 | 265486 | 124567 | 46.9203649 | 5 | 227395 | 77089 | 33.9009213 |
| 6 | 227431 | 73396 | 32.2717659 | 6 | 200725 | 64147 | 31.9576535 |
| 7 | 269259 | 118707 | 44.0865486 | 7 | 231616 | 66886 | 28.8779704 |
| 8 | 301765 | 124054 | 41.1094726 | 8 | 251903 | 77327 | 30.6971334 |
| 9 | 223558 | 78105 | 34.9372422 | 9 | 231841 | 91515 | 39.4731734 |
| 10 | 248844 | 74858 | 30.0823006 | 10 | 277689 | 112275 | 40.431922 |
| | | Average | 41.0627243 | | | Average | 31.9744777 |

Analysis of the planimetry results revealed that the mean decrease in extent of necrosis in treated animals was 22%.

The visual appearance of the flaps was also inspected. Figures 1a-j show control rats 1-10 on day 7 and Figures 2a-j show treatment rats 1-10 on day 7. The treatment rats had received LB-100.

As shown in the photographs of the flaps on day 7 (Figures 1a-j and 2a-j), there was less extensive necrosis at the distal ends of the flap in those animals receiving LB-100.

### Discussion

It has been known since the early 1970s (Maroko et al, 1971) that brief episodes of oxygen deprivation (ischemia) and relief of the ischemia (reperfusion) protects the heart against damage from a subsequent episode. In 1986, Murray et al showed that several short periods of cardiac ischemia reduced the size of tissue damage (infarct) following prolonged ischemia in a canine model. In 2003, Zhao et al. demonstrated that several rounds of ischemia and the return of blood flow (reperfusion) following the induction of an experimental heart attack, also reduced the size of the infarct, compared to simple reperfusion after the attack. The mechanism(s) responsible for cardiac protection by pre- or post- brief cycles of ischemia has been the subject of more than 2500 papers and, although the phenomenon is demonstrable in several animal models, remains unclear. Despite a lack of understanding of the molecular basis of pharmacologic cardioprotection, however, recently there have been increasing calls for finding a way to reduce the amount of cardiac tissue damage following a heart attack (Cohen and Downey, 2011).

In 1998, Weinbrenner et al. reported that fostriecin (FOS), an inhibitor of protein phosphatase 2A, minimizes the size of infarction of cardiac tissue (myocardium) before and even when given after the onset of oxygen deprivation. The following year, Barancik et al. (1999) showed that another known inhibitor of serine/threonine phosphatase, okadaic acid, protected pig myocardium against ischemia (reduced the size of infracted tissue) in an *in vivo* model and also showed that the activity of phosphatases, especially that of PP2A, were decreased in the heart tissue infused with the inhibitor.

FOS, a well studied inhibitor of PP2A, reduces injury even when given after the onset of coronary artery restriction and confirmation of this phenomenon by Barancik et al. (1999) using another PP2A inhibitor, okadaic acid in a pig heart model. Weinbrenner et al. (1998) studied a rabbit and pointed out that death of tissue in the rabbit heart progresses about 5 times faster than in the primate heart, leading them to speculate that administration of a PP2A inhibitor such as FOS as late as 50 minutes after the start of heart attack signs in humans might be expected to offer protection. In earlier studies, Armstrong et al (Armstrong et al. 1997; Armstrong et al. 1998) demonstrated that protein phosphatase inhibitor calyculin A, a PP1/2A inhibitor and FOS both afforded protection from injury to rabbit and pig cells in vitro. Subsequently, Fenton et al. (2005) in rats showed that okadaic acid reduced cellular death following induced MI in both young and aged animals. Fan et al. (2010) studied the effects of okadaic acid at concentrations inhibiting only PP2A and the effects of cantharadin, a naturally occurring inhibitor of both PP1 and PP2A, in isolated perfused functioning rat hearts and concluded that the use of phosphatase inhibitors may provide an approach to reducing reperfusion-induced cell death.

The mechanism by which PP2A confers protection of myocardial damage due to oxygen deprivation is believed to be via activation of a major cell signaling pathway, the PI3K-Akt pathway (Matsui et al. 2001). Recently, Kunuthur (Kunuthur et al. 2011) showed that of three Akt isoforms, Akt1 is essential for cardioprotection against ischemic-reperfusion injury.

Inhibition of PP2A by the novel inhibitors LB-100 and LB-102 and other structural homologs of these compounds have been shown to result in increased phosphorylation of Akt (Lu et al. 2009; US Patent No. 8,0858,268). Phosphorylation of Akt leads to its activation, which in turn increases the phosphorylation of several proteins affecting mitochondrial function and mediating cell death (Tsang et al. 2005).

Without wishing to be bound by any scientific theory, Akt mediates protection by phosphorylation of a number of target proteins, including GSK-3β, endothelial nitric oxide synthase (eNOS), the proapoptotic Bcl-2 family member BAD, caspase 9, the ubiquitin ligase murine double minute 2 (mdm2), and others (Fayard, E. et al, 2005). Phosphorylation of BAD suppresses apoptosis and promotes cell survival (Datta, S.R. et al. 1997) . Overexpression of Akt blocks hypoxia-mediated activation of caspase 9, also blocking their proapoptotic roles (Uchiyama, T. et al. 2004). Akt phosphorylates and activates the ubiquitin ligase, mdm2, which has been shown to play a role in reducing hypoxia-reoxygenation cell death in myocytes (Toth, A. 2006). Akt phosphorylates and activates eNOS, resulting in an increase in nitric oxide (NO) production, which may activate a number of pathways resulting in cardiprotection (Tong, H. et al.). Akt also phosphorylates and inactivates GSK-3β, which also provides an anti-apoptotic effect (Tong et al. 2000).

LB-100, LB-102, and LB-107 and other structural homologs are effective inhibitors of PP2A. LB-102, for example, inhibits PP2A with IC50 of about 0.4uM and to much lesser extent PP1 with an IC50 of about 8.0 uM (Lu et al. 2009(a)). LB-100 is currently entering a Phase I clinical trial in which the compound is anticipated to enhance the cytotoxicity of DNA damaging agents. Although the maximum tolerated dose in humans has not yet been determined, toxicokinetic studies in rats and dogs indicate that the compound can be given with acceptable and reversible toxicity at doses known to inhibit PP2A in tissue of rodents. Thus, LB-100, LB-102 and/or structural homologs can be safely administered to human beings to minimize the extent of myocardial damage following MI. Administration of PP2A is also expected to reduce the extent of tissue damage caused by ischemia in other disease states, such as stroke and acute tissue injury due to trauma, either accidental or surgical injury that compromises blood supply to tissue acutely.

Tissue ischemia also results from hypotension secondary to acute bacterial infections. PP2A is activated due to the inflammatory processes, particularly sepsis, and treatment with LB-100 and structural homologs may be highly beneficial, indeed lifesaving. Sepsis leads to activation of NADPH oxidase and uncoupling of endothelial nitric oxide synthase to produce superoxide, increased NO production and neuronal NOS activity, with increased 3-nitrotyrosine formation and increase PP2A activity in the hind limbs of animals (Zhou et al. 2012). Zhou demonstrated that rapid injection of ascorbate protected against these effects including reduction of PP2A activation. This same mechanism was found by Ladurner (Ladurner et al. 2012). Ladurner showed that in addition to ascorbate, the protein phosphatase inhibitor okadaic acid had similar effects supporting the hypothesis that endothelial damage in these model systems is mediated by PP2A. Han (Han et al. 2010) had previously demonstrated that okadaic acid at a concentration that inhibits PP2A activity decreases endothelial barrier disruption caused by septic insult. Wu and Wilson (Wu et al. 2009) studied mouse skeletal muscle endothelial cells and showed that PP2A inhibition by okadaic acid preserved endothelial barrier function. Thus, LB-100 is expected to be useful in the treatment of conditions such as septic shock in which PP2A mediates vascular leakage.

One mechanism by which fostriecin and the LB-100 compounds reduce tissue damage following acute trauma to tissue may be by inhibition of PP2A in the vasculature. The ability of LB-100 to improve vascular integrity and to reduce tissue damage following acute trauma to tissue was tested by analyzing survival of tissue at the distal end of a flap graft as described in Example 6. The data from Example 6 show that the mean decrease in extent of necrosis in treated animals was 22%. Such an improvement in graft survival would be valuable clinically where necrosis of the distal end of a flap graft is a major limitation to this surgical intervention to correct tissue defects.

### References

Armstrong SC et al (1997) Comparison of in vitro pre-conditioning responses of isolated pig and rabbit cardiomyocytes: effects of a protein phosphatase inhibitor fostriecin. J Mol Cell Cardiol 29:3009-3024.
Armstrong SC et al (1998) Protein phosphates inhibitors calyculin A and fostriecin protect rabbit cardiomyocytes in late ischemia. J Mol Cell Cardiol 30:61-73.
Barancik M et al (1999) Okadaic acid and anisomycin are protective and stimulate the SAPK/JNK pathway. Journal of Cardiovascular Pharmacology 34(2):182-190.
Bell, R. et al. (2011) There is More to Life than Revascularization: Therapeutic Targeting of Myocardial ischemia/Reperfusion Injury. Cardiovascular Therapeutics, 29, e67-e79.
Cohen MV and Downey JM (2011) Is it time to translate ischemic preconditioning's mechanism of cardioprotection into clinical practice? J of Cardiovascular Pharmacology and Therapeutics 16(3-4) :273-280.
Datta, S.R. (1997) Akt Phosphorylation of BAD Couples Survival Signals to the Cell-Intrinsic Death Machinery. Cell, 91, 231-241.
Fan WJ (2010) Kinases and phosphatases in ischaemic preconditioning: a re-evaluation. Basic RES Cardiol 105:495-511. Fenton et al (2005). Inhibition of phosphatase activity enhances pre-conditioning and limits cell death in the ischemic/reperfused aged rat heart. Life Sciences 77:3375-3388.
Fayard E, Tintignac LA, Baudry A, Hemmings BA. (2005) Protein kinase B/Akt at a glance. J Cell Sci 118: 5675-5678.
Kunuthur SP et al. (2011) The Akt1 isoform is an essential mediator of ischemic preconditioning. Journal of Cellular and Molecular Medicine. "Accepted Article"; doi:10.1111/j.1582-4934.2011.01491.x
Ladurner et al. (2012) Ascorbate stimulates endothelial nitric oxide synthese enzyme activity by rapid modulation of its phosphorylation status. Free Radical Biology and Medicine, In Press.
Longacre LS et al (2011) New horizons in cardio protection. Circulation 124:1172-1179.
Lu J et al (2009) The effect of a PP2A inhibitor on the nuclear receptor corepressor pathway in glioma. Journal of Neurosurgery DOI:10.3171/2009.11 JNS091272.
Lu J et al (2009)(a) Inhibition of serine/threonine phosphatase PP2A enhances cancer chemotherapy by blocking DNA damage induced defense mechanisms. PNAS 106(28):11697-11702.
Maroko PR et al (1971) Factors influencing infarct size following experimental coronary artery occlusions. Circulation 43:67-82.
Matsui T et al (2001). Akt activation preserves cardiac function and prevents injury after transient carciac ischemia in vivo. Circulation 104:330-335.
USPTO 8,058,268 B2. Neuroprotective agents for the prevention and treatment of neurodegenerative diseases.
Tsang A et al. (2005) Myocardial postconditioning: reperfusion injury revisited. American Journal of Physiology Heart Circulation Physiology 289: H2-H7.
Tong H, Chen W, Steenbergen C, Murphy E. (2000) Ischemic preconditioning activates phosphatidylinositol-3-kinase upstream of protein kinase C. Circ Res 87: 309-315.
Toth A, Nickson P, Qin LL, Erhardt P. (2006) Differential regulation of cardiomyocyte survival and hypertrophy by MDM2, an E3 ubiquitin ligase. J Biol Chem 281: 3679-3689.
Uchiyama T, Engelman RM, Maulik N, Das DK. (2004) Role of Akt signaling in mitochondrial survival pathway triggered by hypoxic preconditioning. Circulation 109: 3042-3049.
Weinbrenner C et al (1998) Fostriecin, an inhibitor of phosphatase 2A, limits myocardial infarct size even when administered after onset of ischemia. Circulation 98:899-905.
Wu, et al. (2007) Activation or Protein Phosphatase 2A by Palmitate Inhibits AMP-activated Protein Kinase. The Journal of Biological Chemisty. 282, 13, 9777-9788.
Yellon DM and Hausenloy DJ (2005) Realizing the clinical potential of ischemic preconditioning and postconditioning. Nature Clinical Practice Cardiovascular Medicine 2(11)568-575.
Zhou, G. et al. (2012) Ascorbate protects against vascular leakage in cecal ligation and puncture-induced septic peritonitis. American J Physiol Regul Integr Comp Physiol 302:409-416.
Zhao Z-Q et al (2003) Inhibition of myocardial injury by ischemic postconditioning during reperfusion: comparison with preconditioning. American Journal of Physiology 285(2):H579-H588.

## Claims

1. A protein phosphatase 2A (PP2A) inhibitor having the structure: 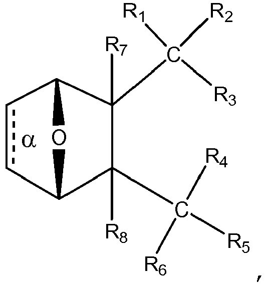 wherein
bond α is present or absent;
R₁ and R₂ is each independently H, O⁻ or OR₉,
where R₈ is H, alkyl, alkenyl, alkynyl or aryl,
or R₁ and R₂ together are =O;
R₃ and R₄ are each different, and each is OH, O⁻, OR₉, O(CH₂)₁₋₆R₉, SH, S⁻, SR₉, 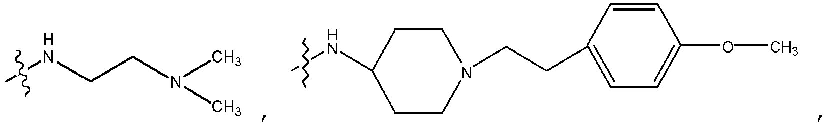 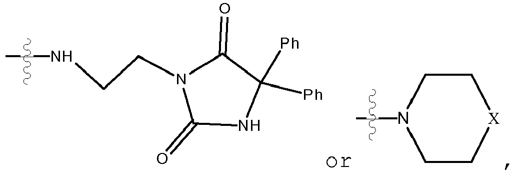 where X is O, S, NR₁₀, or N⁺R₁₀R₁₀,
where each R₁₀ is independently H, alkyl, C₂-C₁₂ alkyl, alkenyl, C₄-C₁₂ alkenyl, alkynyl, aryl, substituted aryl where the substituent is other than chloro when R₁ and R₂ are =O, 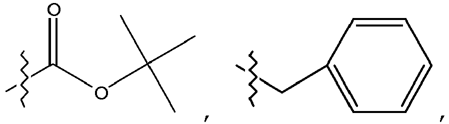 -CH₂CN, -CH₂CO₂R₁₁, -CH₂COR₁₁, -NHR₁₁ or -NH⁺(R₁₁)₂,
wherein each R₁₁ is independently alkyl, alkenyl or alkynyl, or H;
R₅ and R₆ is each independently H, OH, or R₅ and R₆ taken together are =O;
R₇ and R₈ is each independently H, F, Cl, Br, SO₂Ph, CO₂CH₃, or SR₁₂,
where R₁₂ is H, alkyl, alkenyl, alkynyl or aryl; and
each occurrence of alkyl, alkenyl, or alkynyl is branched or unbranched, unsubstituted or substituted,
or a salt, enantiomer or zwitterion of the compound, for use in reducing reperfusion injury in mammalian tissue in a subject or for use in reducing tissue damage due to an acute trauma in a subject or for use in reducing vascular leakage in a subject due to an acute trauma.

2. The inhibitor for use according to claim 1 having the structure: 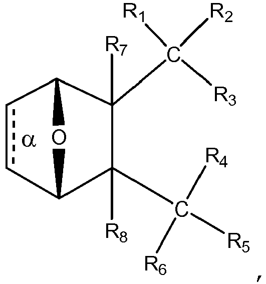 wherein
bond a is present or absent;
R₁ and R₂ is each independently H, O⁻ or OR₉,
where R₈ is H, alkyl, alkenyl, alkynyl or aryl,
or R₁ and R₂ together are =O;
R₃ and R₄ are each different, and each is OH, O⁻, OR₉, O(CH₂)₁₋₆R₉, SH, S⁻, SR₉, 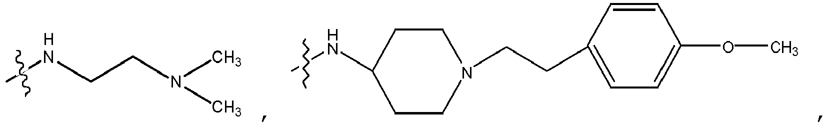 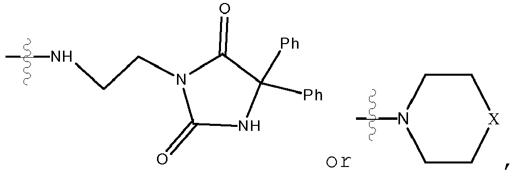 where X is O, S, NR₁₀, or N⁺R₁₀R₁₀,
where each R₁₀ is independently H, alkyl, C₂-C₁₂ alkyl, alkenyl, C₄-C₁₂ alkenyl, alkynyl, aryl, substituted aryl where the substituent is other than chloro when R₁ and R₂ are =O, 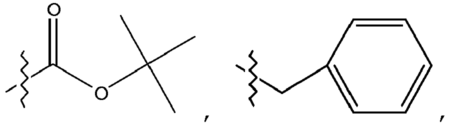 -CH₂CN, -CH₂CO₂R₁₁, -CH₂COR₁₁, -NHR₁₁ or -NH⁺(R_{N})₂,
wherein each R₁₁ is independently alkyl, alkenyl or alkynyl, or H;
R₅ and R₆ is each independently H, OH, or R₅ and R₆ taken together are =O;
R₇ and R₈ is each independently H, F, Cl, Br, SO₂Ph, CO₂CH₃, or SR₁₂,
where R₁₂ is H, alkyl, alkenyl, alkynyl or aryl; and
each occurrence of alkyl, alkenyl, or alkynyl is branched or unbranched, unsubstituted or substituted,
or a salt, enantiomer or zwitterion of the compound, for use in reducing reperfusion injury in mammalian tissue in a subject.

3. The inhibitor for use according to claim 2, wherein the reduction of reperfusion injury comprises increased phosphorylation of Akt in the mammalian tissue that has suffered an ischemia, or wherein the reduction of reperfusion injury comprises increased activation of Akt in the mammalian tissue that has suffered an ischemia, or wherein the reduction of reperfusion injury comprises increased phosphorylation of BAD, mdm2, eNOS and/or GSK-3β in the mammalian tissue that has suffered an ischemia.

4. The inhibitor for use according to claim 3, wherein the ischemia is caused by a myocardial infarction, stroke or sepsis.

5. The inhibitor for use according to any one of claims 2-4, wherein the tissue is myocardial tissue, brain tissue or endothelial tissue.

6. The inhibitor for use according to any one of claims 1-5, wherein the protein phosphatase 2A inhibitor has the structure 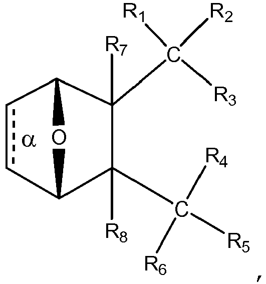 wherein
bond α is present or absent;
R₁ and R₂ is each independently H, O⁻ or OR₉,
where R₉ is H, alkyl, alkenyl, alkynyl or aryl,
or R₁ and R₂ together are =O;
R₃ and R₄ are each different, and each is OH, O⁻, OR₉, SH, S⁻, SR₉, 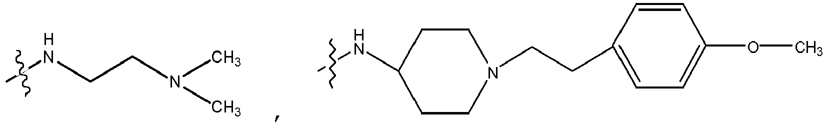 or 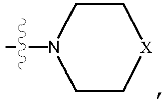 where X is O, S, NR₁₀, or N⁺R₁₀R₁₀,
where each R₁₀ is independently H, alkyl, C₂-C₁₂ alkyl, alkenyl, C₄-C₁₂ alkenyl, alkynyl, aryl, substituted aryl where the substituent is other than chloro when R₁ and R₂ are =O, 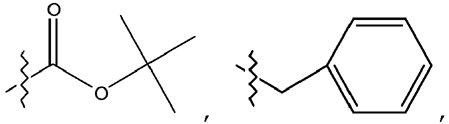 -CH₂CN, -CH₂CO₂R₁₁, -CH₂COR₁₁, -NHR₁₁ or -NH⁺(R₁₁)₂,
where each R₁₁ is independently alkyl, alkenyl alkynyl, or H;
R₅ and R₆ is each independently H, OH, or R₅ and R₆ taken together are =O;
R₇ and R₈ is each independently H, F, Cl, Br, SO₂Ph, CO₂CH₃, or SR₁₂,
where R₁₂ is H, aryl or a substituted or unsubstituted alkyl, alkenyl or alkynyl; and
each occurrence of alkyl, alkenyl, or alkynyl is branched or unbranched, unsubstituted or substituted,
or a salt, enantiomer or zwitterion of the compound.

7. The inhibitor for use according to claim 6,
wherein
R₁ and R₂ together are =O;
R₃ is O⁻ or OR₉,
where R₉ is H, methyl, ethyl or phenyl;
R₄ is 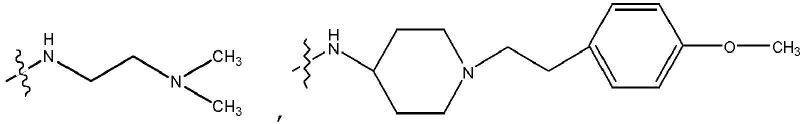 or 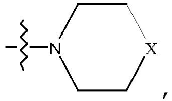 where X is O, S, NR₁₀, or N⁺R₁₀R₁₀,
where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro, 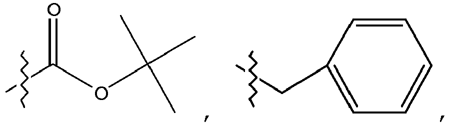 -CH₂CN, -CH₂CO₂R₁₁, -CH₂COR₁₁, -NHR₁₁ or -NH⁺(R₁₁)₂,
where R₁₁ is alkyl, alkenyl or alkynyl, each of which is substituted or unsubstituted, or H;
R₅ and R₆ taken together are =O; and
R₇ and R₈ is each independently H, F, Cl, Br, SO₂Ph, CO₂CH₃, or SR₁₂,
where R₁₂ is a substituted or unsubstituted alkyl, alkenyl or alkynyl.

8. The inhibitor for use according to claim 6 or 7, wherein R₄ is 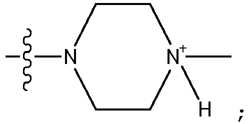 or wherein R₄ is 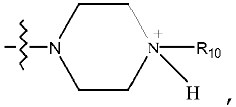 where R₁₀ is 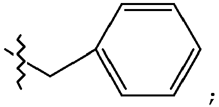 or wherein R₄ is 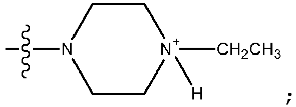 or wherein R₄ is

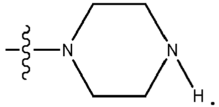

9. The inhibitor for use according to claim 6, wherein the protein phosphatase 2A inhibitor has the structure 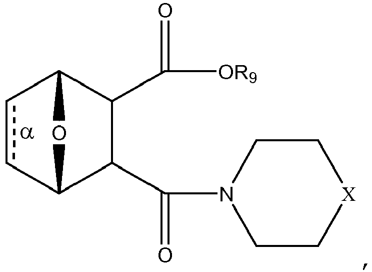 wherein
bond α is present or absent;
R₉ is present or absent and when present is H, alkyl, alkenyl, alkynyl or phenyl; and
X is O, NR₁₀, or N⁺R₁₀R₁₀,
where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro, 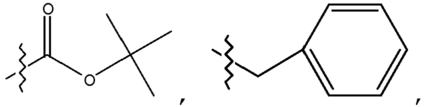 -CH₂CN, -CH₂CO₂R₁₂, or -CH₂COR₁₂,
where R₁₂ is H or alkyl,
or a salt, zwitterion, or enantiomer of the compound;
or wherein the protein phosphatase 2A inhibitor has the structure 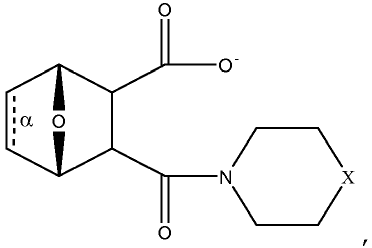 wherein
bond α is present or absent;
X is O or NH⁺R₁₀,
where R₁₀ is H, alkyl, substituted C₂-C₁₂ alkyl, alkenyl, substituted C₄-C₁₂ alkenyl, alkynyl, substituted alkynyl, aryl, substituted aryl where the substituent is other than chloro, 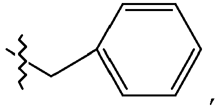 -CH₂CN, -CH₂CO₂R₁₂, or -CH₂COR₁₂,
where R₁₂ is H or alkyl; or
wherein the protein phosphatase 2A inhibitor has the structure 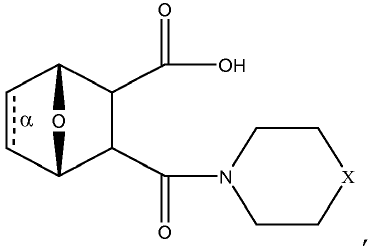 wherein
bond α is present or absent; X is NH⁺R₁₀,
where R₁₀ is present or absent and when present R₁₀ is alkyl, substituted C₂-C₁₂ alkyl, alkenyl, substituted C₄-C₁₂ alkenyl, 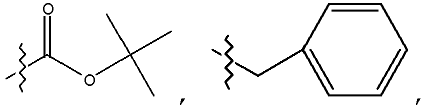 -CH₂CN, -CH₂CO₂R₁₂, or -CH₂COR₁₂,
where R₁₂ is H or alkyl.

10. The inhibitor for use according to claim 6, wherein the protein phosphatase 2A inhibitor has the structure 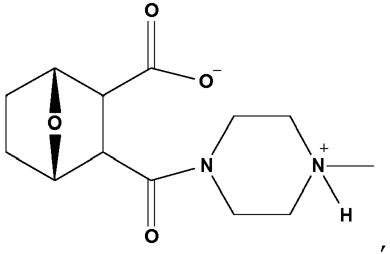 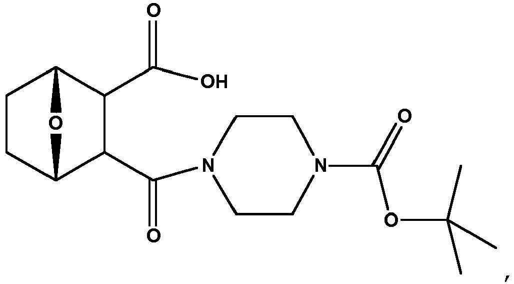 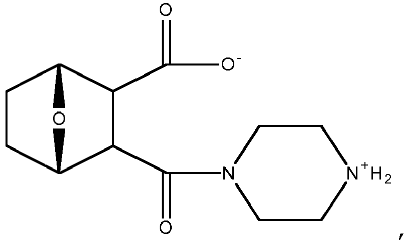 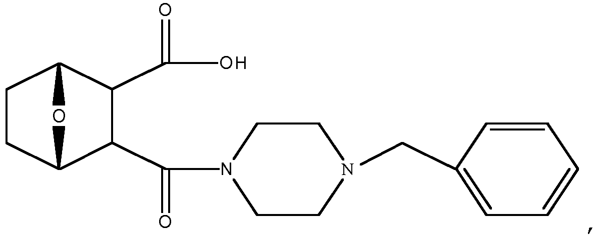 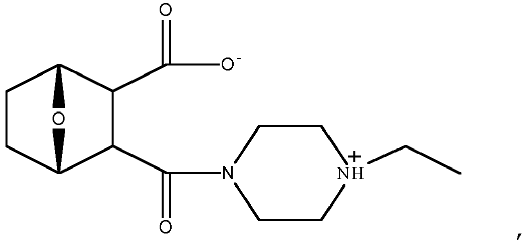 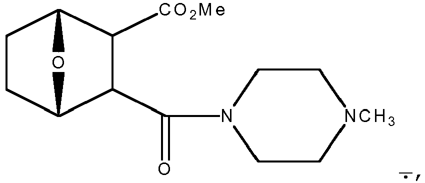 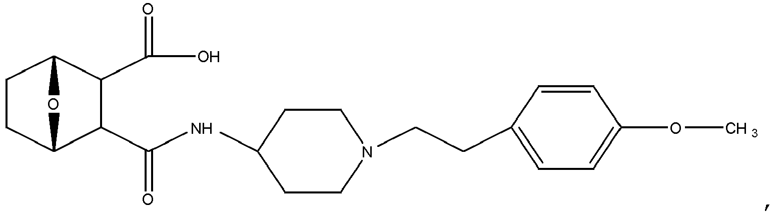 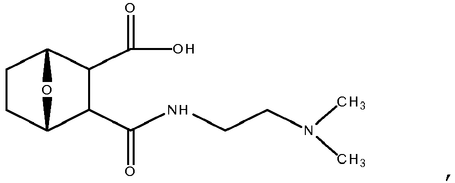 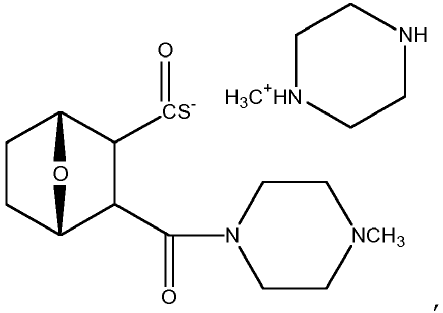 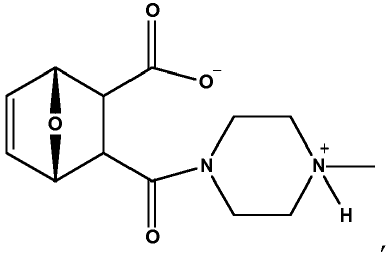 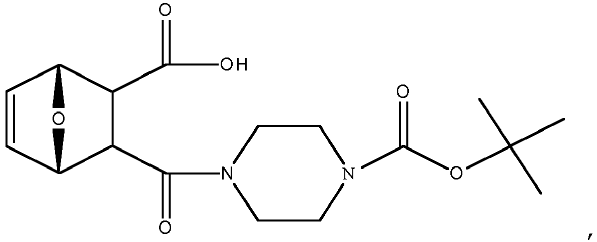 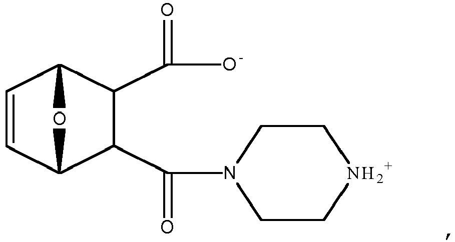 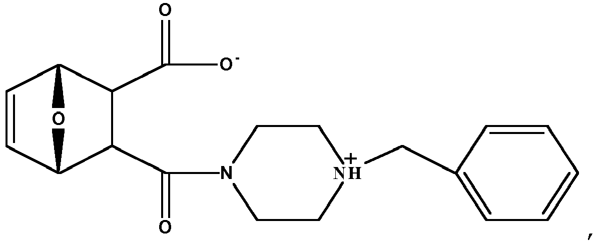 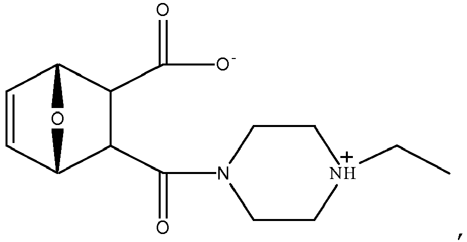 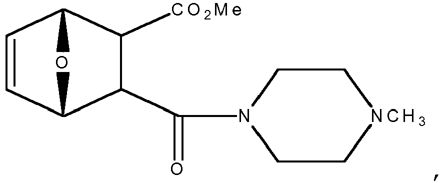 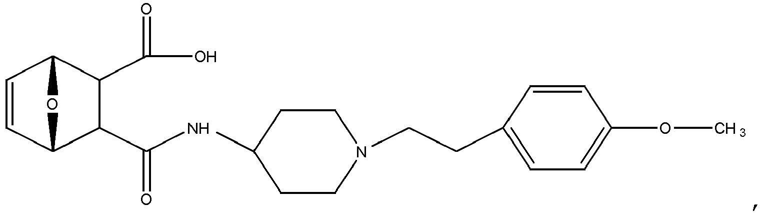 or

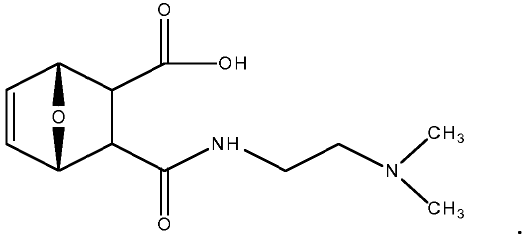

11. The inhibitor for use according to any one of claims 1-5, wherein the protein phosphatase 2A inhibitor has the structure 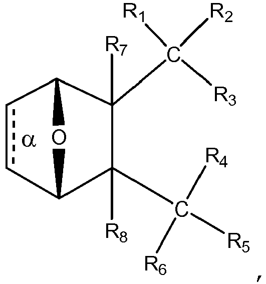 wherein
bond α is present or absent;
R₁ and R₂ is each independently H, O⁻ or OR₉,
where R₉ is H, alkyl, alkenyl, alkynyl or aryl,
or R₁ and R₂ together are =O;
R₃ and R₄ are each different, and each is O(CH₂)₁₋₆R₈ or OR₉, or 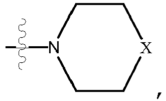 where X is O, S, NR₁₀, or N⁺R₁₀R₁₀,
where each R₁₀ is independently H, alkyl, hydroxyalkyl, C₂-C₁₂ alkyl, alkenyl, C₄-C₁₂ alkenyl, alkynyl, aryl, where the substituent is other than chloro when R₁ and R₂ are =O, 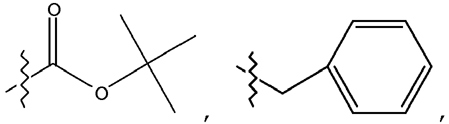 -CH₂CN, -CH₂CO₂R₁₁, -CH₂COR₁₁, -NHR₁₁ or -NH⁺(R₁₁)₂,
where each R₁₁ is independently H, alkyl, alkenyl, or alkynyl;
or R₃ and R₄ are each different and each is OH or 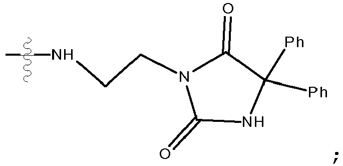 R₅ and R₆ is each independently H, OH, or R₅ and R₆ taken together are =O;
R₇ and R₈ is each independently H, F, Cl, Br, SO₂Ph, CO₂CH₃, or SR₁₂,
where R₁₂ is H, aryl or a substituted or unsubstituted alkyl, alkenyl or alkynyl; and
each occurrence of alkyl, alkenyl, or alkynyl is branched or unbranched, unsubstituted or substituted,
or a salt, enantiomer or zwitterion of the compound.

12. The inhibitor for use according to claim 11,
wherein
R₁ and R₂ together are =O;
R₃ is OR₉ or O(CH₂)₁₋₂R₉,
where R₉ is aryl, substituted ethyl, or substituted phenyl, wherein the substituent is in the para position of the phenyl;
or R₃ is OH and R₄ is 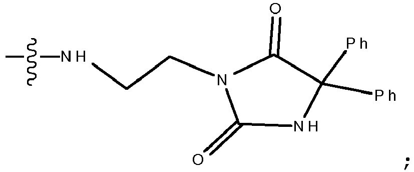 R₄ is 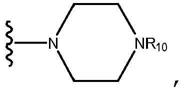 where R₁₀ is alkyl or hydroxyl alkyl;
R₅ and R₆ together are =O; and
R₇ and R₈ are each independently H, or wherein
R₁ and R₂ together are =O;
R₃ is OH, O(CH₂)R₉, or OR₉,
where R₉ is phenyl or CH₂CCl₃, 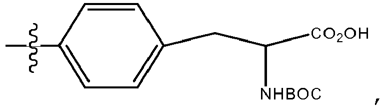 or 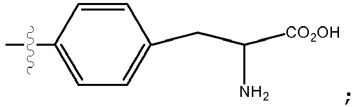 R₄ is 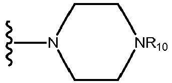 or 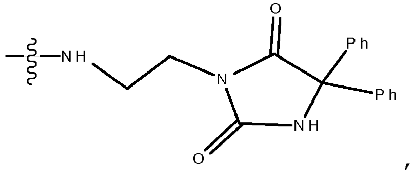 where R₁₀ is CH₃ or CH₃CH₂OH;
R₅ and R₆ together are =O; and
R₇ and R₈ are each independently H, or
wherein R₃ is OR₉
where R₉ is (CH₂)₁₋₆(CHNHBOC)CO₂H, (CH₂)₁₋₆(CHNH₂)CO₂H,
(CH₂)₁₋₆CCl₃, CH₂(CHNHBOC) CO₂H, CH₂(CHNH₂)CO₂H, or CH₂CCl₃.

13. The inhibitor for use according to claim 11, wherein the protein phosphatase 2A inhibitor has the structure 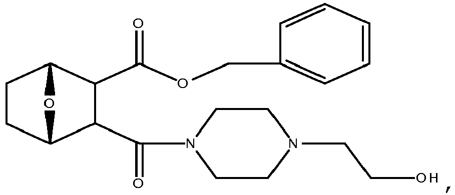 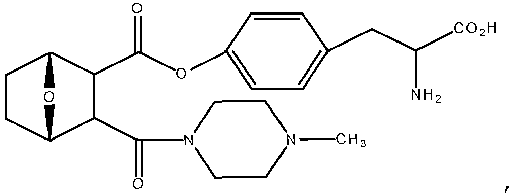 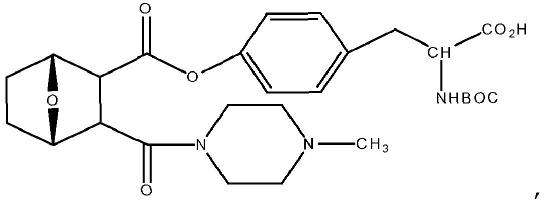 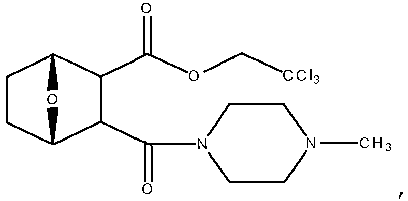 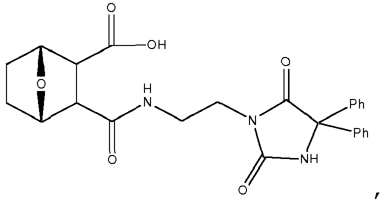 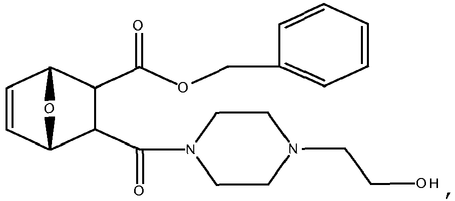 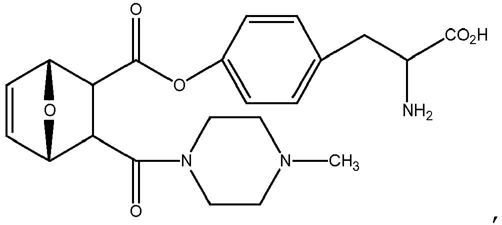 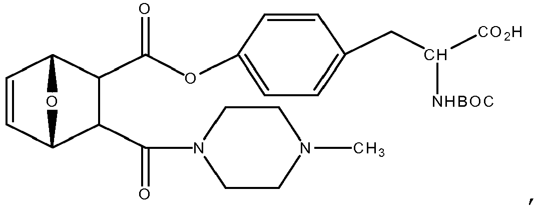 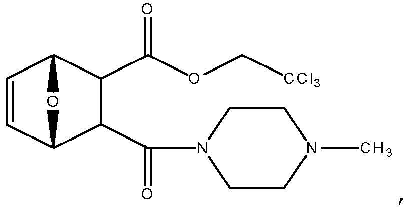 or

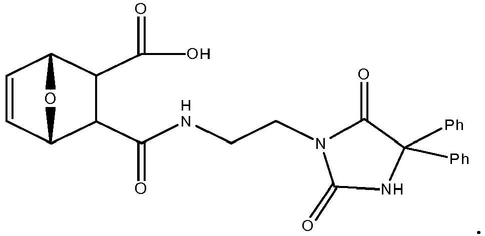

14. The inhibitor for use according to claim 4, wherein tissue damage associated with reperfusion injury in the heart of the subject following a myocardial infarction is reduced; or wherein vascular leakage associated with reperfusion injury in a subject suffering from sepsis is reduced.

15. The inhibitor for use according to claim 1, wherein the reperfusion injury is tissue damage associated with reperfusion injury in the heart of the subject following myocardial infarction and the tissue is myocardial tissue.

16. The inhibitor for use according to claim 1, wherein the reperfusion injury is tissue damage due to an acute trauma in the subject and the tissue is endothelial tissue.

## Patentansprüche

1. Ein Proteinphosphatase 2A (PP2A) -Inhibitor, der die Struktur hat: 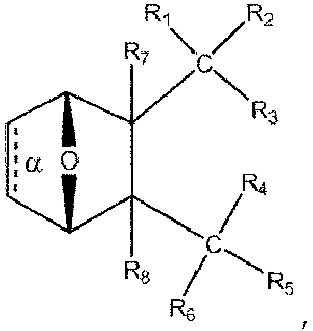 wobei
Bindung α ist vorhanden oder nicht vorhanden;
R₁ und R₂ ist jeweils unabhängig H, O⁻, oder OR₉,
wobei R₉ ist H, Alkyl, Alkenyl, Alkinyl, oder Aryl,
oder R₁ und R₂ zusammen sind =O;
R₃ und R₄ sind jeweils verschieden, und jedes ist OH, O⁻, OR₉, O(CH₂)₁₋₆R₉, SH, S⁻, SR₉, 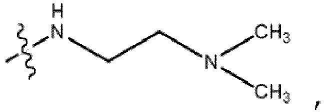 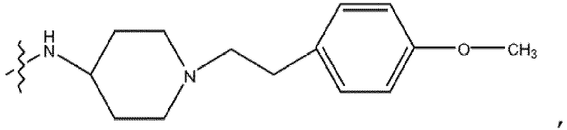 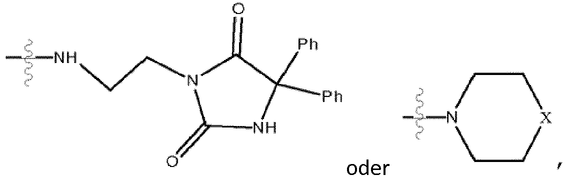 wobei X ist O, S, NR₁₀ oder N⁺R₁₀R₁₀,
wobei jedes R₁₀ ist unabhängig H, Alkyl, C₂-C₁₂ Alkyl, Alkenyl, C₄-C₁₂ Alkenyl, Alkinyl, Aryl, substituiertes Aryl
wobei der Substituent ein anderer als Chlor ist, wenn R₁ und R₂ sind =O, 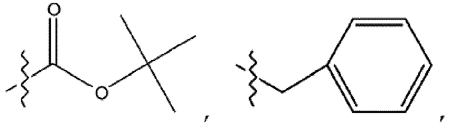 -CH₂CN, - CH₂CO₂R₁₁, -CH₂COR₁₁, - NHR₁₁ oder -NH⁺(R₁₁)₂
wobei jedes R₁₁ ist unabhängig Alkyl, Alkenyl, oder
Alkinyl, oder H;
R₅ und R₆ ist jeweils unabhängig H, OH oder R₅ und R₆ zusammengenommen sind =O;
R₇ und R₈ ist jeweils unabhängig H, F, Cl, Br, SO₂Ph, CO₂CH₃ oder SR₁₂,
wobei R₁₂ ist H, Alkyl, Alkenyl, Alkinyl, oder Aryl; und
jedes vorhandene Alkyl, Alkenyl oder Alkinyl ist verzweigt oder unverzweigt, unsubstituiert oder substituiert,
oder ein Salz, Enantiomer oder Zwitterion der Verbindung, zur Verwendung in der Reduzierung von Reperfusionsverletzungen in Säugetiergewebe in einem Subjekt oder zur Verwendung für die Reduzierung von Gewebeschaden aufgrund von akutem Trauma in einem Subjekt oder zur Verwendung für die Reduzierung von Gefäßundichtigkeit in einem Subjekt aufgrund von akutem Trauma.

2. Der Inhibitor zur Verwendung gemäß Anspruch 1, der die Struktur hat: 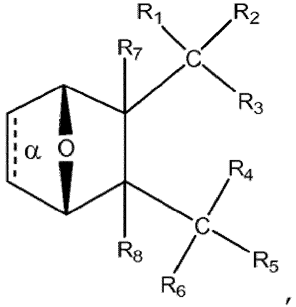 wobei
Bindung α ist vorhanden oder nicht vorhanden;
R₁ und R₂ ist jeweils unabhängig H, O⁻, oder OR₉,
wobei R₉ ist H, Alkyl, Alkenyl, Alkinyl, oder Aryl,
oder R₁ und R₂ zusammen sind =O;
R₃ und R₄ sind jeweils verschieden, und jedes ist OH, O⁻, OR₉, O(CH₂)₁₋₆R₉, SH, S⁻, SR₉, 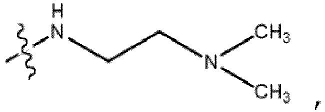 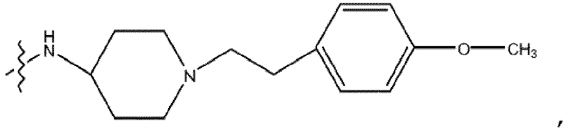 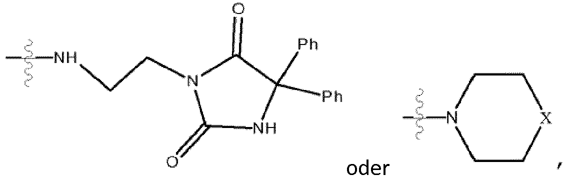 wobei X ist O, S, NR₁₀ oder N⁺R₁₀R₁₀,
wobei jedes R₁₀ ist unabhängig H, Alkyl, C₂-C₁₂ Alkyl, Alkenyl, C₄-C₁₂ Alkenyl, Alkinyl, Aryl, substituiertes Aryl
wobei der Substituent ein anderer als Chlor ist, wenn R₁ und R₂ sind =O, 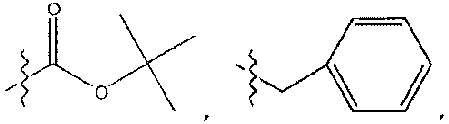 -CH₂CN, - CH₂CO₂R₁₁, -CH₂COR₁₁, - NHR₁₁ oder -NH⁺(R₁₁)₂
wobei jedes R₁₁ ist unabhängig Alkyl, Alkenyl, oder
Alkinyl, oder H;
R₅ und R₆ ist jeweils unabhängig H, OH oder R₅ und R₆ zusammengenommen sind =O;
R₇ und R₈ ist unabhängig H, F, Cl, Br, SO₂Ph, CO₂CH₃ oder SR₁₂,
wobei R₁₂ ist H, Alkyl, Alkenyl, Alkinyl, oder Aryl; und
jedes vorhandene Alkyl, Alkenyl oder Alkinyl ist verzweigt oder unverzweigt, unsubstituiert oder substituiert,
oder ein Salz, Enantiomer oder Zwitterion der Verbindung, zur Verwendung für die Reduzierung von Reperfusionsverletzung in Säugetiergewebe in einem Subjekt.

3. Der Inhibitor zur Verwendung gemäß Anspruch 2, wobei die Reduzierung von Reperfusionsverletzung die erhöhte Phosphorylierung von Akt im Säugetiergewebe das an Ischämie gelitten hat umfasst, oder wobei die Reduzierung von Reperfusionsverletzung die erhöhte Aktivierung von Akt im Säugetiergewebe, das an Ischämie gelitten hat, umfasst, oder wobei die Reduzierung von Reperfusionsverletzung die erhöhte Phosphorylierung von BAD, mdm2, eNOS und/oder GSK-3β im Säugetiergewebe, das an Ischämie gelitten hat, umfasst.

4. Der Inhibitor zur Verwendung gemäß Anspruch 3, wobei die Ischämie durch einen Herzinfarkt, Schlaganfall oder Sepsis verursacht wird.

5. Der Inhibitor zur Verwendung gemäß einem der Ansprüche 2 bis 4, wobei das Gewebe Herzmuskelgewebe, Gehirngewebe oder Endothelgewebe ist.

6. Der Inhibitor zur Verwendung gemäß einem der Ansprüche 1-5, wobei der Proteinphosphatase 2A -Inhibitor die Struktur hat 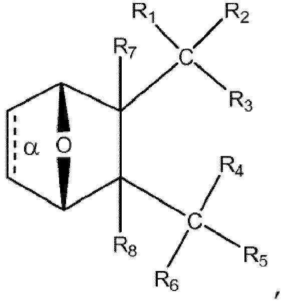 wobei
Bindung α ist vorhanden oder nicht vorhanden;
R₁ und R₂ ist jeweils unabhängig H, O⁻, oder OR₉,
wobei R₉ ist H, Alkyl, Alkenyl, Alkinyl, oder Aryl,
oder R₁ und R₂ zusammen sind =O;
R₃ und R₄ sind jeweils verschieden, und jedes ist OH, O⁻, OR₉, SH, S⁻, SR₉, 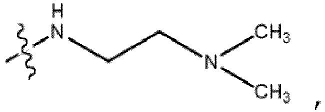 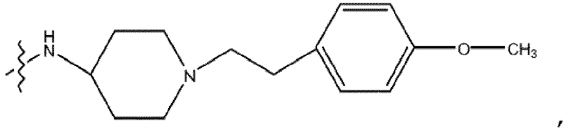 oder 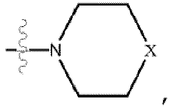 wobei X ist O, S, NR₁₀ oder N⁺R₁₀R₁₀,
wobei jedes R₁₀ ist unabhängig H, Alkyl, C₂-C₁₂ Alkyl, Alkenyl, C₄-C₁₂ Alkenyl, Alkinyl, Aryl, substituiertes Aryl
wobei der Substituent ein anderer als Chlor ist, wenn R₁ und R₂ sind =O, 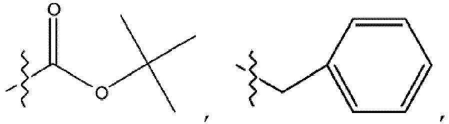 -CH₂CN, - CH₂CO₂R₁₁, -CH₂COR₁₁, - NHR₁₁ oder -NH⁺(R₁₁)₂
wobei jedes R₁₁ ist unabhängig Alkyl, Alkenyl,
Alkinyl, oder H;
R₅ und R₆ ist jeweils unabhängig H, OH oder R₅ und R₆ zusammengenommen sind =O; R₇ und R₈ ist unabhängig H, F, Cl, Br, SO₂Ph, CO₂CH₃ oder SR₁₂,
wobei R₁₂ ist H, Aryl oder substituiertes oder unsubstituiertes
Alkyl, Alkenyl oder Alkinyl; und
jedes auftretende Alkyl, Alkenyl oder Alkinyl ist verzweigt oder unverzweigt, unsubstituiert oder substituiert,
oder ein Salz, Enantiomer oder Zwitterion der Verbindung.

7. Der Inhibitor zur Verwendung gemäß Anspruch 6,
wobei
R₁ und R₂ zusammengenommen sind =O;
R₃ ist O⁻ oder OR₉,
wobei R₉ ist H, Methyl oder Ethyl oder Phenyl;
R₄ ist 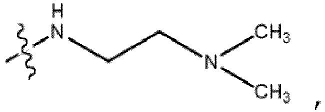 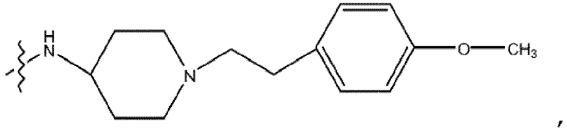 oder 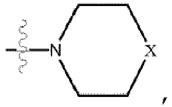 wobei X ist O, S, NR₁₀ oder N⁺R₁₀R₁₀,
wobei jedes R₁₀ ist unabhängig H, Alkyl, substituiertes C₂-C₁₂ Alkyl, Alkenyl, substituiertes C₄-C₁₂ Alkenyl, Alkinyl, substituiertes Alkinyl, Aryl, substituiertes Aryl wobei der Substituent ein anderer als Chlor ist, 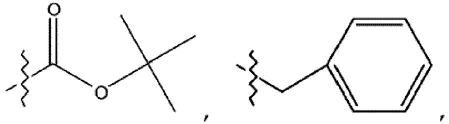 -CH₂CN, -CH₂CO₂R₁₁, -CH₂COR₁₁, -NHR₁₁ oder -NH⁺(R₁₁)₂,
wobei R₁₁ ist Alkyl, Akenyl oder Alkinyl, jeweils substituiert oder unsubstiuiert oder H;
R₅ und R₆ zusammengenommen sind =O; und
R₇ und R₈ ist jeweils unabhängig H, F; Cl, Br, SO₂Ph, CO₂CH₃ oder SR₁₂,
wobei R₁₂ ist ein substituiertes oder unsubstituiertes Alkyl, Alkenyl oder Alkinyl.

8. Der Inhibitor zur Verwendung gemäß Anspruch 6 oder 7, wobei R₄ ist 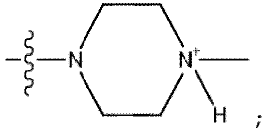 oder wobei R₄ ist 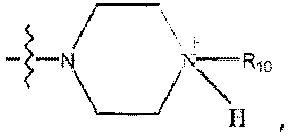 wobei R₁₀ ist 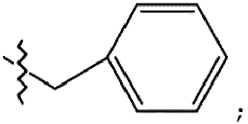 oder wobei R₄ ist 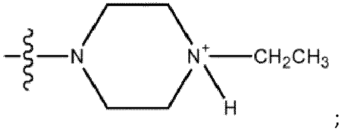 oder wobei R₄ ist

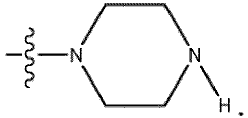

9. Der Inhibitor zur Verwendung gemäß Anspruch 6, wobei der Proteinphosphatase 2A - Inhibitor die Struktur hat 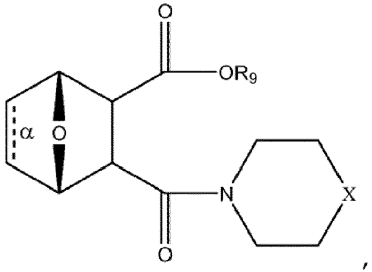 wobei
Bindung α ist vorhanden oder nicht vorhanden;
R₉ ist vorhanden oder nicht vorhanden und wenn vorhanden ist H, Alkyl, Alkenyl, Alkinyl oder Phenyl; und
X ist O, NR₁₀ oder N⁺R₁₀R₁₀,
wobei jedes R₁₀ ist unabhängig H, Alkyl, substituiertes C₂-C₁₂ Alkyl, Alkenyl, substituiertes C₄-C₁₂ Alkenyl, Alkinyl, substituiertes Alkinyl, Aryl, substituiertes Aryl wobei der Substituent ein anderer ist als Chlor, 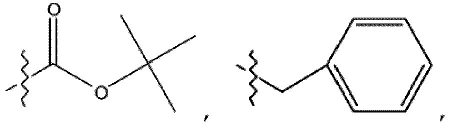 -CH₂CN, -CH₂CO₂R₁₂ oder -CH₂COR₁₂,
wobei R₁₂ ist H oder Alkyl,
oder ein Salz, Zwitterion oder Enantiomer der Verbindung;
oder wobei der Proteinphosphatase 2A -Inhibitor die Struktur hat 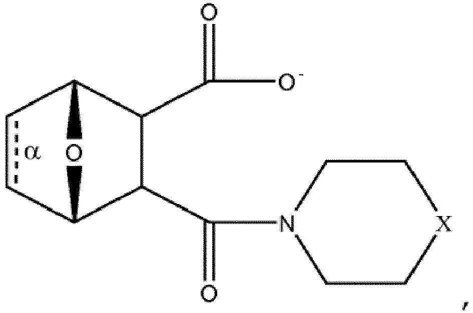 wobei
Bindung α ist vorhanden oder nicht vorhanden;
X ist O oder NH⁺R₁₀,
wobei R₁₀ ist H, Alkyl, substituiertes C₂-C₁₂ Alkyl, Alkenyl, substituiertes C₄-C₁₂ Alkenyl, Alkinyl, substituiertes Alkinyl, Aryl, substituiertes Aryl wobei der Substituent ein anderer ist als Chlor, 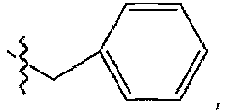 -CH₂CN, -CH₂CO₂R₁₂, oder -CH₂COR₁₂,
wobei R₁₂ ist H oder Alkyl; oder
wobei der Proteinphosphatase 2A -Inhibitor die Struktur hat 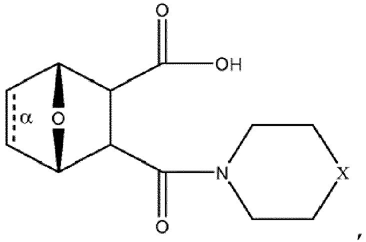 wobei
Bindung α ist vorhanden oder nicht vorhanden; X ist NH⁺R₁₀,
wobei R₁₀ ist vorhanden oder nicht vorhanden und wenn vorhanden R₁₀ ist Alkyl, substituiertes C₂-C₁₂ Alkyl, Alkenyl, substituiertes C₄-C₁₂ Alkenyl, 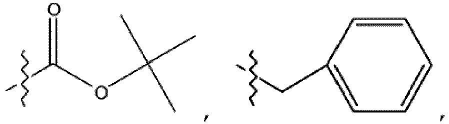 -CH₂CN, -CH₂CO₂R₁₂ oder -CH₂COR₁₂,
wobei R₁₂ ist H oder Alkyl.

10. Der Inhibitor zur Verwendung gemäß Anspruch 6, wobei der Proteinphosphatase 2A - Inhibitor die Struktur hat 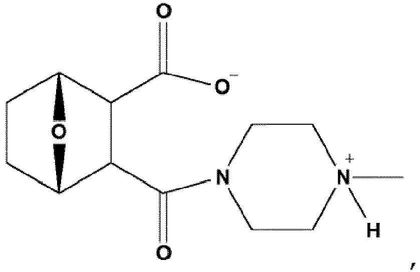 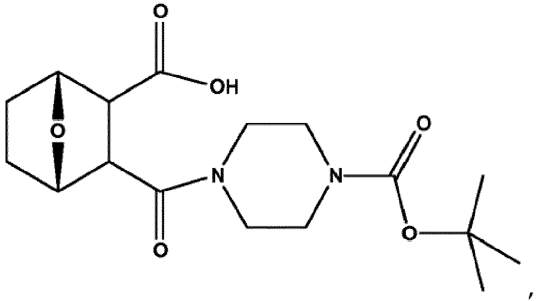 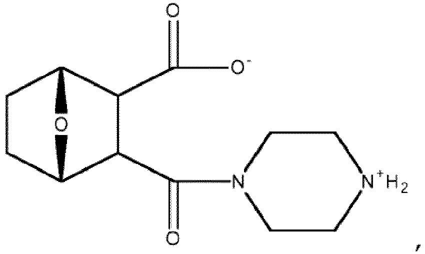 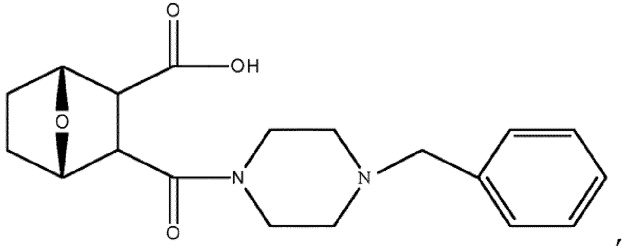 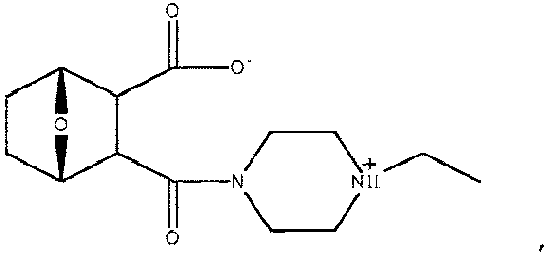 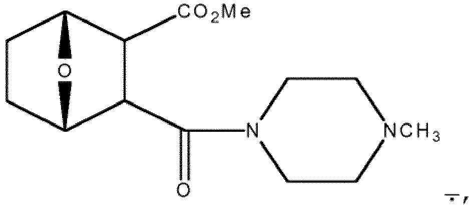 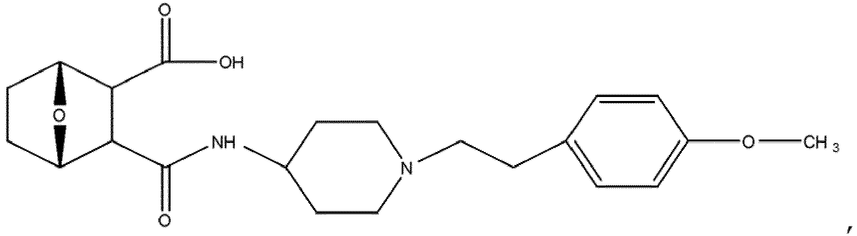 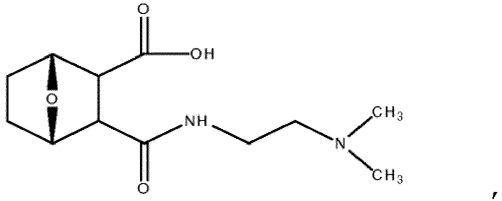 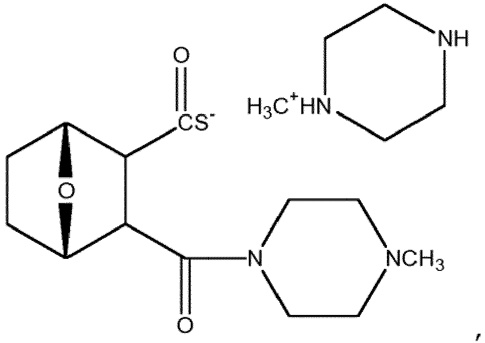 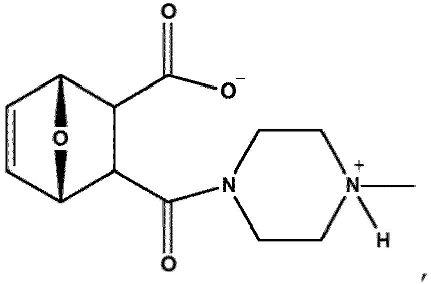 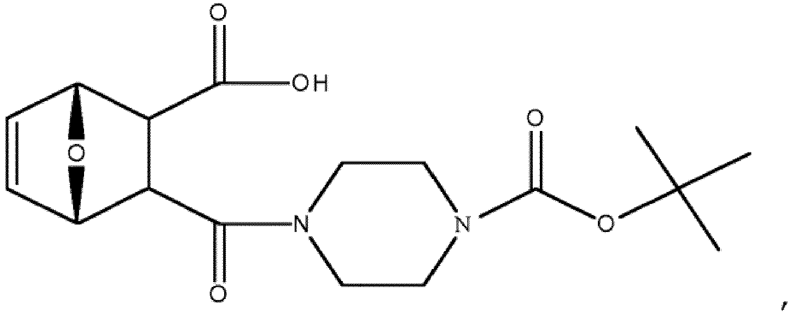 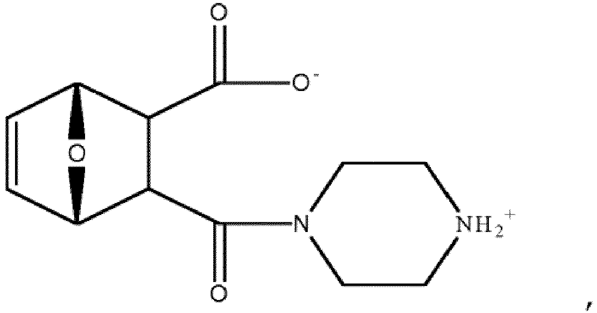 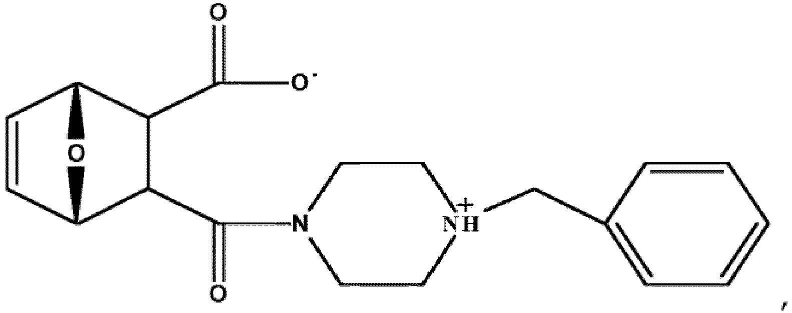 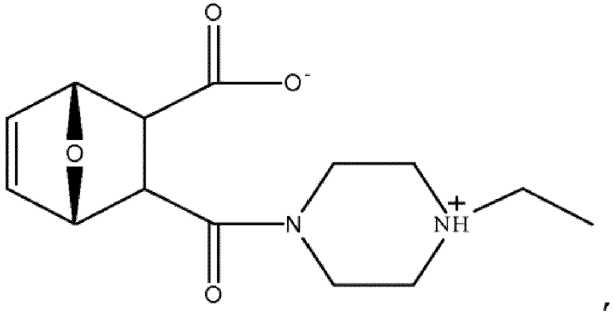 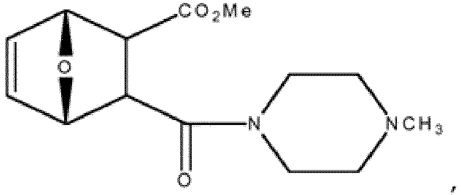 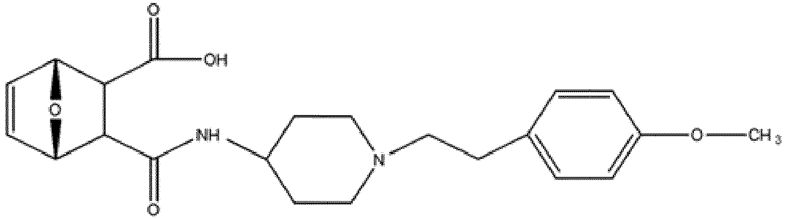 oder

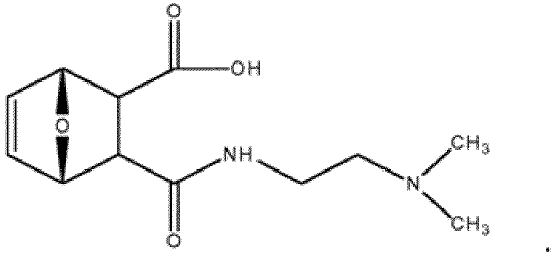

11. Der Inhibitor zur Verwendung gemäß einem der Ansprüche 1-5, wobei der Proteinphosphatase 2A -Inhibitor die Struktur hat 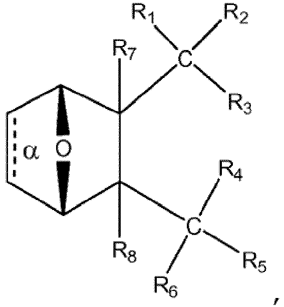 wobei
Bindung α ist vorhanden oder nicht vorhanden;
R₁ und R₂ ist jeweils unabhängig H, O⁻ oder OR₉,
wobei R₉ ist H, Alkyl, Alkenyl, Alkinyl oder Aryl,
oder R₁ und R₂ zusammengenommen sind =O;
R₃ und R₄ sind jeweils verschieden und jeweils O(CH₂)₁₋₆R₉ oder OR₉ oder 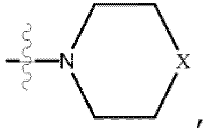 wobei X ist O, S, NR₁₀ oder N⁺R₁₀R₁₀,
wobei jedes R₁₀ ist unabhängig H, Alkyl, Hydroxyalkyl, C₂-C₁₂ Alkyl, Alkenyl, C₄-C₁₂ Alkenyl, Alkinyl, Aryl, wobei der Substituent ein anderer ist als Chlor wenn R₁ und R₂ sind =O, 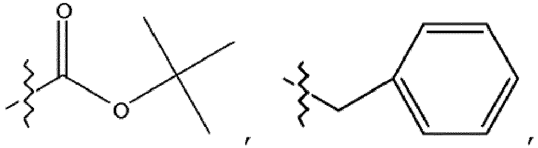 -CH₂CN, -CH₂CO₂R₁₁, -CH₂COR₁₁, -NHR₁₁, oder -NH⁺(R₁₁)₂,
wobei jedes R₁₁ ist unabhängig H, Alkyl,
Alkenyl oder Alkinyl;
oder R₃ und R₄ sind jeweils verschieden und sind jeweils OH oder 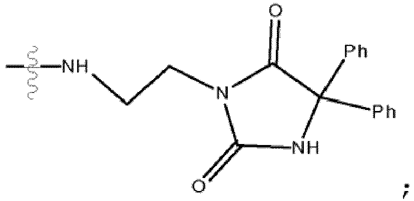 R₅ und R₆ sind jeweils unabhängig H, OH oder R₅ und R₆ zusammengenommen sind =O;
R₇ und R₈ ist jeweils unabhängig H, F, Cl, Br, SO₂Ph, CO₂CH₃ oder SR₁₂,
wobei R₁₂ ist H, Aryl oder ein substituiertes oder unsubstituiertes Alkyl, Alkenyl oder Alkinyl; und jedes vorhandene Alkyl, Alkenyl oder Alkinyl ist verzweigt oder unverzweigt, unsubstituiert oder substituiert,
oder ein Salz, Enantiomer oder Zwitterion der Verbindung.

12. Der Inhibitor zur Verwendung gemäß Anspruch 11,
wobei,
R₁ und R₂ zusammengenommen sind =O;
R₃ ist OR₉ oder O(CH₂)₁₋₂R₉,
wobei R₉ ist Aryl, substituiertes Ethyl oder substituiertes
Phenyl, wobei der Substituent ist in der Paraposition des Phenyls;
oder R₃ ist OH und R₄ ist 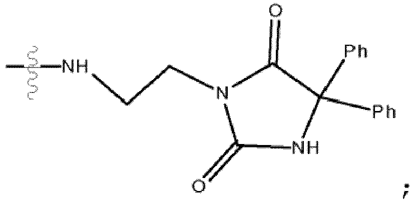 R₄ ist 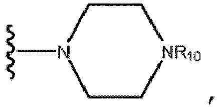 wobei R₁₀ ist Alkyl oder Hydroxyalkyl ;
R₅ und R₆ zusammengenommen sind =O; und
R₇ und R₈ sind jeweils unabhängig H, oder
wobei
R₁ und R₂ zusammengenommen sind =O;
R₃ ist OH, O(CH₂)R₉ oder OR₉,
wobei R₉ ist Phenyl oder CH₂CCl₃, 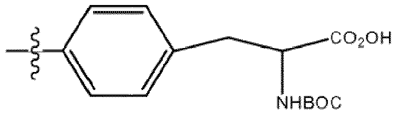 oder 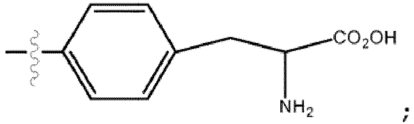 R₄ ist 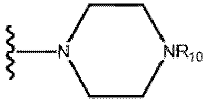 oder 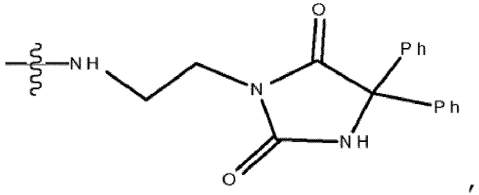 wobei R₁₀ ist CH₃ oder CH₃CH₂OH;
R₅ und R₆ zusammengenommen sind =O; und
R₇ und R₈ sind jeweils unabhängig H, oder
wobei R₃ ist OR₉
wobei R₉ ist (CH₂)₁₋₆(CHNHBOC)CO₂H, (CH₂)₁₋₆(CHNH₂)CO₂H,
(CH₂)₁₋₆CCl₃, CH₂(CHNHBOC)CO₂H, CH₂(CHNH₂)CO₂H oder CH₂CCl₃.

13. Der Inhibitor zur Verwendung gemäß Anspruch 11, wobei der Proteinphosphatase 2A - Inhibitor die Struktur hat 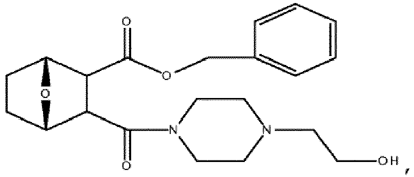 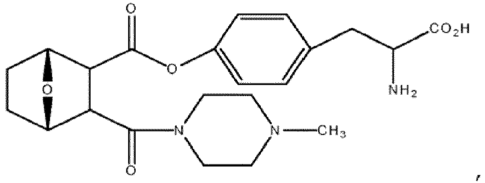 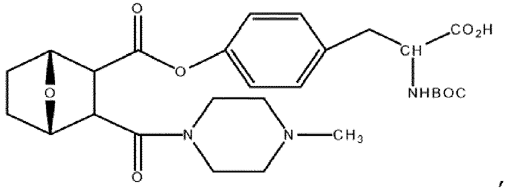 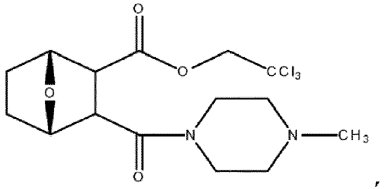 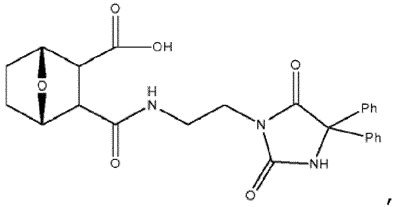 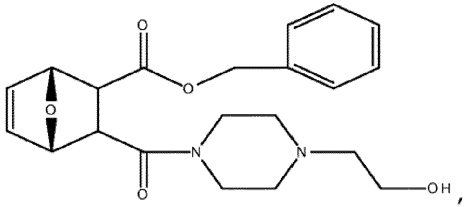 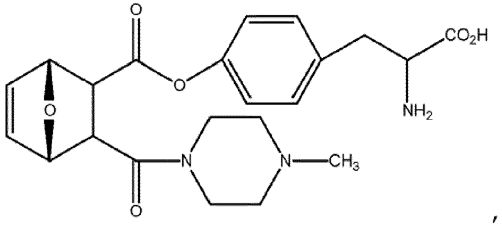 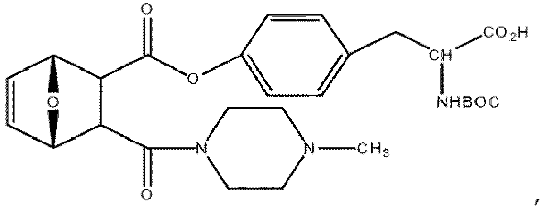 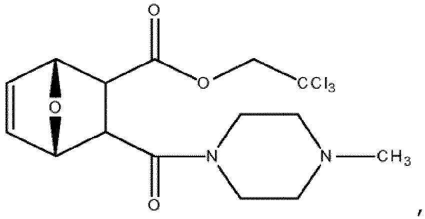 oder

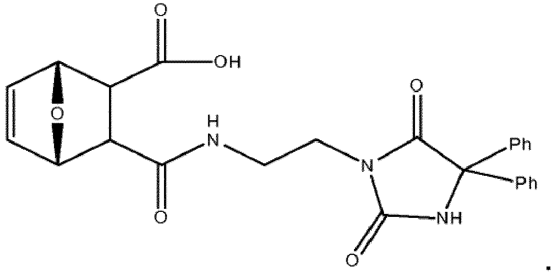

14. Der Inhibitor zur Verwendung gemäß Anspruch 4, wobei Gewebeschaden, assoziiert mit einer Reperfusionsverletzung im Herzen des Subjekts, die auf einen Herzinfarkt folgt, reduziert ist; oder wobei Gefäßundichtigkeit assoziiert mit Reperfusionsverletzung in einem Subjekt, das an Sepsis leidet, reduziert ist.

15. Der Inhibitor zur Verwendung gemäß Anspruch 1, wobei die Reperfusionsverletzung Gewebeschaden, assoziiert mit einer Reperfusionsverletzung im Herzen des Subjekts folgend auf einen Herzinfarkt und das Gewebe Myocardgewebe ist.

16. Der Inhibitor zur Verwendung gemäß Anspruch 1, wobei die Reperfusionsverletzung Gewebeschaden aufgrund eines akuten Traumas in dem Subjekt und das Gewebe Endothelgewebe ist.

## Revendications

1. Inhibiteur de la protéine phosphatase 2A (PP2A) ayant la structure : 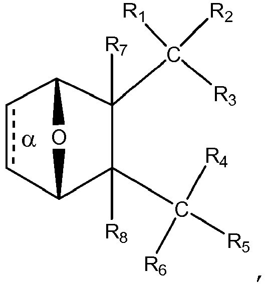 dans lequel
la liaison α est présente ou absente ;
R₁ et R₂ représentent chacun indépendamment H, O⁻ ou OR₉,
où R₉ représente H, un groupe alkyle, alcényle, alcynyle ou aryle,
ou R₁ et R₂ ensemble représentent =O ;
R₃ et R₄ sont chacun différents, et chacun représente OH, O⁻, OR₉, O(CH₂)₁₋₆R₉, SH, S⁻, SR₉, 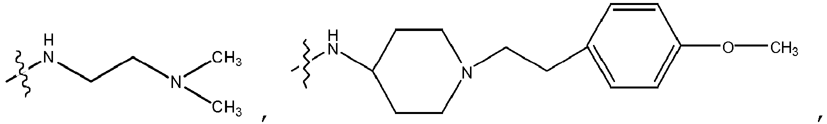 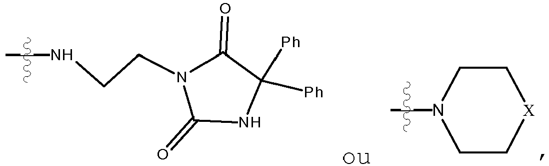 où X représente O, S, NR₁₀, ou N⁺R₁₀R₁₀,
où chaque R₁₀ représente indépendamment H, un groupe alkyle, alkyle en C₂ à C₁₂, alcényle, alcényle en C₄ à C₁₂, alcynyle, aryle, aryle substitué où le substituant est autre qu'un groupe chloro lorsque R₁ et R₂ représentent =O, 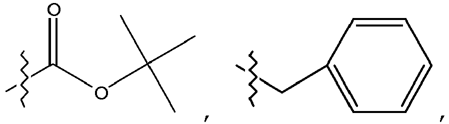 -CH₂CN, -CH₂CO₂R₁₁, -CH₂COR₁₁, -NHR₁₁ ou -NH⁺(R₁₁)₂,
où chaque R₁₁ représente indépendamment un groupe alkyle, alcényle ou alcynyle, ou H ;
R₅ et R₆ représentent chacun indépendamment H, OH, ou R₅ et R₆ pris ensemble représentent =O ;
R₇ et R₈ représentent chacun indépendamment H, F, Cl, Br, SO₂Ph, CO₂CH₃, ou SR₁₂,
où R₁₂ représente H, un groupe alkyle, alcényle, alcynyle ou aryle ; et
chaque occurrence des groupes alkyle, alcényle, ou alcynyle est ramifiée ou non ramifiée, non substituée ou substituée,
ou un sel, un énantiomère ou un zwittérion du composé, destiné à être utilisé dans la réduction de la lésion de reperfusion dans du tissu de mammifère chez un sujet ou destiné à être utilisé dans la réduction des dommages tissulaires dus à un traumatisme aigu chez un sujet ou destiné à être utilisé dans la réduction de la fuite vasculaire chez un sujet due à un traumatisme aigu.

2. Inhibiteur destiné à être utilisé selon la revendication 1 ayant la structure : 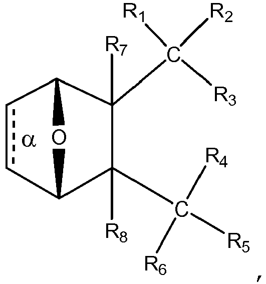 dans lequel
la liaison α est présente ou absente ;
R₁ et R₂ représentent chacun indépendamment H, O⁻ ou OR₉,
où R₉ représente H, un groupe alkyle, alcényle, alcynyle ou aryle,
ou R₁ et R₂ ensemble représentent =O ;
R₃ et R₄ sont chacun différents, et chacun représente OH, O⁻, OR₉, O(CH₂)₁₋₆R₉, SH, S⁻, SR₉, 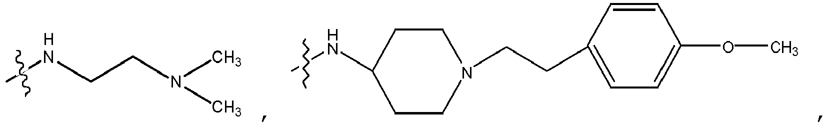 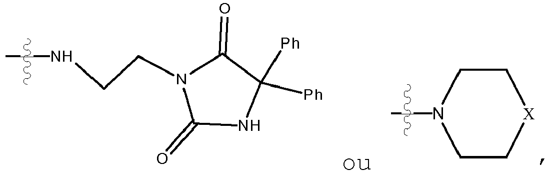 où X représente O, S, NR₁₀, ou N⁺R₁₀R₁₀,
où chaque R₁₀ représente indépendamment H, un groupe alkyle, alkyle en C₂ à C₁₂, alcényle, alcényle en C₄ à C₁₂, alcynyle, aryle, aryle substitué où le substituant est autre qu'un groupe chloro lorsque R₁ et R₂ représentent =O, 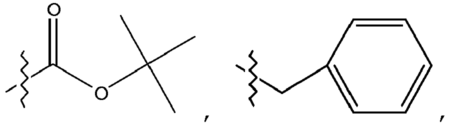 -CH₂CN, -CH₂CO₂R₁₁, -CH₂COR₁₁, -NHR₁₁ ou -NH⁺(R₁₁)₂,
où chaque R₁₁ représente indépendamment un groupe alkyle, alcényle ou alcynyle, ou H ;
R₅ et R₆ représentent chacun indépendamment H, OH, ou R₅ et R₆ pris ensemble représentent =O ;
R₇ et R₈ représentent chacun indépendamment H, F, Cl, Br, SO₂Ph, CO₂CH₃, ou SR₁₂,
où R₁₂ représente H, un groupe alkyle, alcényle, alcynyle ou aryle ; et
chaque occurrence des groupes alkyle, alcényle, ou alcynyle est ramifiée ou non ramifiée, non substituée ou substituée,
ou un sel, un énantiomère ou un zwittérion du composé, destiné à être utilisé dans la réduction de la lésion de reperfusion dans du tissu de mammifère chez un sujet.

3. Inhibiteur destiné à être utilisé selon la revendication 2, dans lequel la réduction de la lésion de reperfusion comprend la phosphorylation augmentée de Akt dans le tissu de mammifère qui a souffert d'une ischémie, ou dans lequel la réduction de la lésion de reperfusion comprend l'activation augmentée de Akt dans le tissu de mammifère qui a souffert d'une ischémie, ou dans lequel la réduction de la lésion de reperfusion comprend une phosphorylation augmentée de BAD, mdm2, eNOS et/ou GSK-3β dans le tissu de mammifère qui a souffert d'une ischémie.

4. Inhibiteur destiné à être utilisé selon la revendication 3, dans lequel l'ischémie est provoquée par un infarctus du myocarde, un accident vasculaire cérébral ou une septicémie.

5. Inhibiteur destiné à être utilisé selon l'une quelconque des revendications 2 à 4, dans lequel le tissu est le tissu myocardique, le tissu cérébral ou le tissu endothélial.

6. Inhibiteur destiné à être utilisé selon l'une quelconque des revendications 1 à 5, dans lequel l'inhibiteur de la protéine phosphatase 2A a la structure 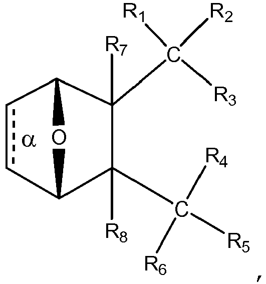 dans lequel
la liaison α est présente ou absente ;
R₁ et R₂ représentent chacun indépendamment H, O⁻ ou OR₉,
où R₉ représente H, un groupe alkyle, alcényle, alcynyle ou aryle,
ou R₁ et R₂ ensemble représentent =O ;
R₃ et R₄ sont chacun différents, et chacun représente OH, O⁻, OR₉, SH, S⁻, SR₉, 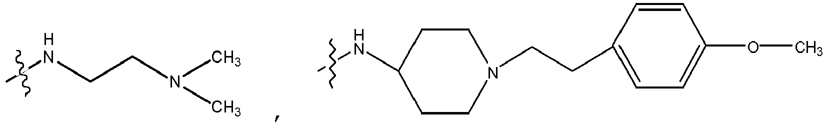 ou 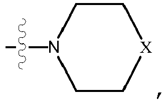 où X représente O, S, NR₁₀, ou N⁺R₁₀R₁₀,
où chaque R₁₀ représente indépendamment H, un groupe alkyle, alkyle en C₂ à C₁₂, alcényle, alcényle en C₄ à C₁₂, alcynyle, aryle, aryle substitué où le substituant est autre qu'un groupe chloro lorsque R₁ et R₂ représentent =O, 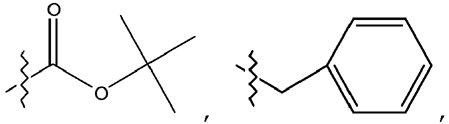 -CH₂CN, -CH₂CO₂R₁₁, -CH₂COR₁₁, -NHR₁₁ ou -NH⁺(R₁₁)₂,
où chaque R₁₁ représente indépendamment un groupe alkyle, alcényle ou alcynyle, ou H ;
R₅ et R₆ représentent chacun indépendamment H, OH, ou R₅ et R₆ pris ensemble représentent =O ;
R₇ et R₈ représentent chacun indépendamment H, F, Cl, Br, SO₂Ph, CO₂CH₃, ou SR₁₂,
où R₁₂ représente H, un groupe aryle, un groupe substitué ou non substitué alkyle, alcényle ou alcynyle ; et
chaque occurrence des groupes alkyle, alcényle, ou alcynyle est ramifiée ou non ramifiée, non substituée ou substituée,
ou un sel, un énantiomère ou un zwittérion du composé.

7. Inhibiteur destiné à être utilisé selon la revendication 6,
dans lequel
R₁ et R₂ ensemble représentent =O ;
R₃ représente O⁻ ou OR₉,
où R₉ représente H, un groupe méthyle, éthyle ou phényle ;
R₄ représente 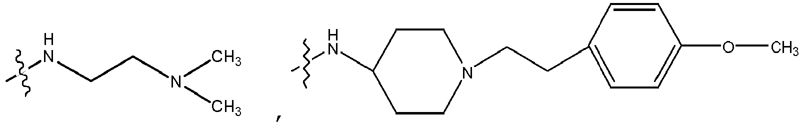 ou 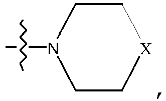 où X représente O, S, NR₁₀, ou N⁺R₁₀R₁₀,
où chaque R₁₀ représente indépendamment H, un groupe alkyle, alkyle en C₂ à C₁₂ substitué, alcényle, alcényle en C₄ à C₁₂ substitué, alcynyle, alcynyle substitué, aryle, aryle substitué où le substituant est autre qu'un groupe chloro, 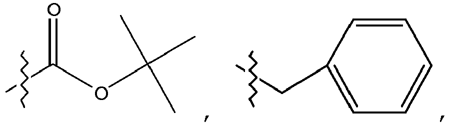 -CH₂CN, -CH₂CO₂R₁₁, -CH₂COR₁₁, -NHR₁₁ ou -NH⁺(R₁₁)₂,
où R₁₁ représente un groupe alkyle, alcényle ou alcynyle, chacun d'entre eux est substitué ou non substitué, ou H ;
R₅ et R₆ pris ensemble représentent =O ; et
R₇ et R₈ représentent chacun indépendamment H, F, Cl, Br, SO₂Ph, CO₂CH₃, ou SR₁₂,
où R₁₂ représente un groupe substitué ou non substitué alkyle, alcényle ou alcynyle.

8. Inhibiteur destiné à être utilisé selon la revendication 6 ou 7, dans lequel R₄ représente 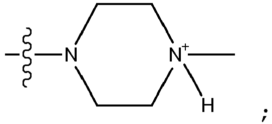 ou
dans lequel R₄ représente 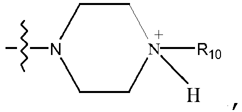 où R₁₀ représente 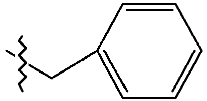 ou
dans lequel R₄ représente 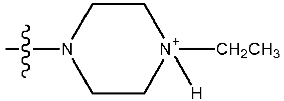 ou
dans lequel R₄ représente

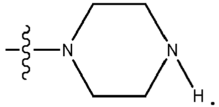

9. Inhibiteur destiné à être utilisé selon la revendication 6, dans lequel l'inhibiteur de la protéine phosphatase 2A a la structure 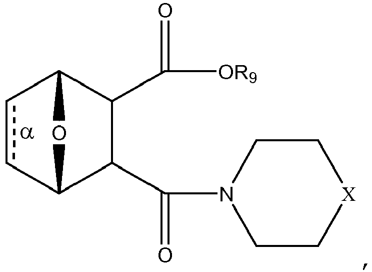 dans lequel
la liaison α est présente ou absente ;
R₉ est présent ou absent et lorsqu'il est présent, représente H, un groupe alkyle, alcényle, alcynyle ou phényle ; et
X représente O, NR₁₀, ou N⁺R₁₀R₁₀,
où chaque R₁₀ représente indépendamment H, un groupe alkyle, alkyle en C₂ à C₁₂ substitué, alcényle, alcényle en C₄ à C₁₂ substitué, alcynyle, alcynyle substitué, aryle, aryle substitué où le substituant est autre qu'un groupe chloro, 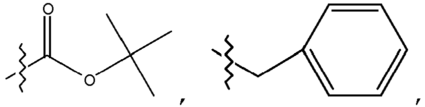 -CH₂CN, -CH₂CO₂R₁₂, ou -CH₂COR₁₂,
où R₁₂ représente H ou un groupe alkyle,
ou un sel, un zwittérion, ou un énantiomère du composé ;
ou dans lequel l'inhibiteur de la protéine phosphatase 2A a la structure 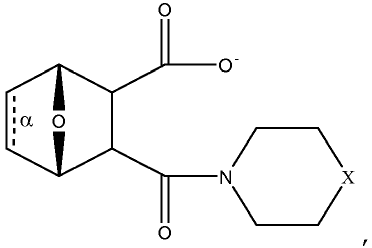 dans lequel
la liaison α est présente ou absente ;
X représente O, ou NH⁺R₁₀,
où R₁₀ représente indépendamment H, un groupe alkyle, alkyle en C₂ à C₁₂ substitué, alcényle, alcényle en C₄ à C₁₂ substitué, alcynyle, alcynyle substitué, aryle, aryle substitué où le substituant est autre qu'un groupe chloro, 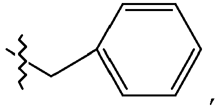 -CH₂CN, -CH₂CO₂R₁₂, ou -CH₂COR₁₂,
où R₁₂ représente H ou un groupe alkyle ; ou
dans lequel l'inhibiteur de la protéine phosphatase 2A a la structure 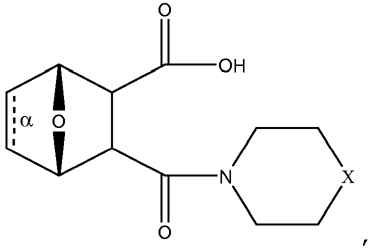 dans lequel
la liaison α est présente ou absente ; X représente NH⁺R₁₀,
où R₁₀ est présent ou absent, et lorsqu'il est présent, R₁₀ représente un groupe alkyle, alkyle en C₂ à C₁₂ substitué, alcényle, alcényle en C₄ à C₁₂ substitué, 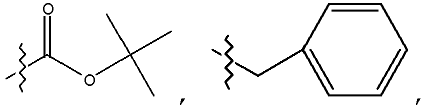 -CH₂CN, -CH₂CO₂R₁₂, ou -CH₂COR₁₂,
où R₁₂ représente H ou un groupe alkyle.

10. Inhibiteur destiné à être utilisé selon la revendication 6, dans lequel l'inhibiteur de la protéine phosphatase 2A a la structure 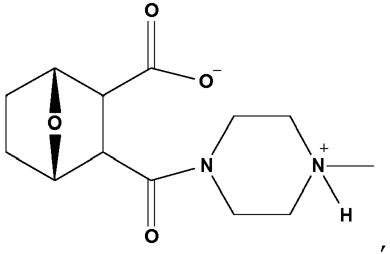 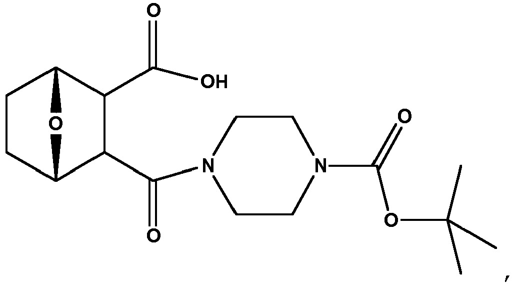 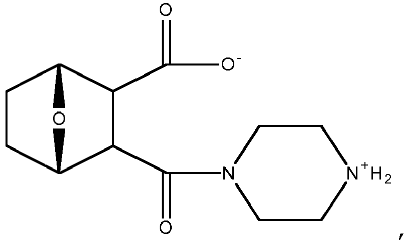 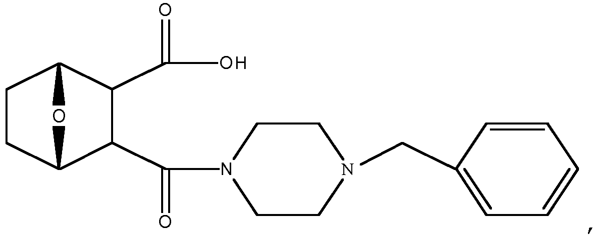 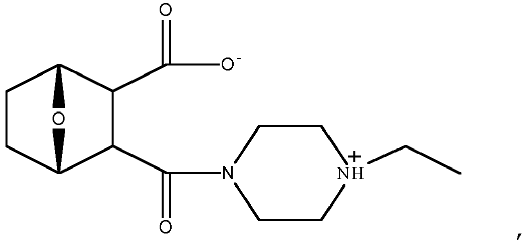 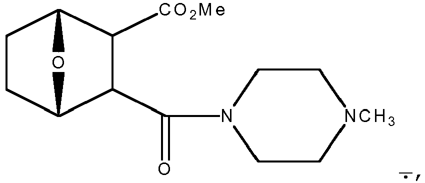 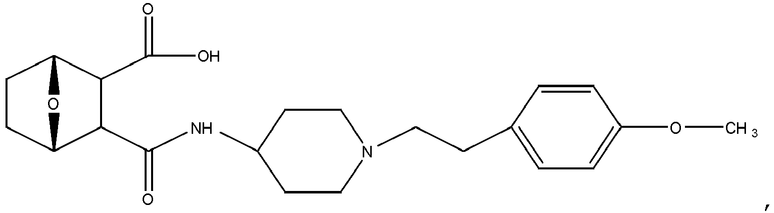 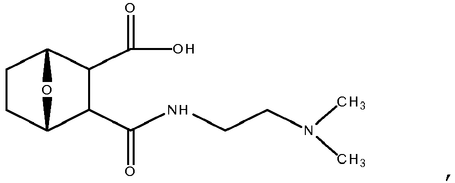 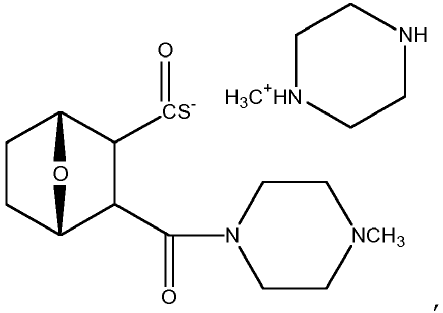 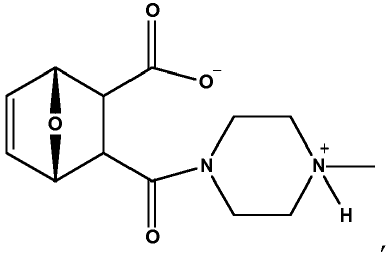 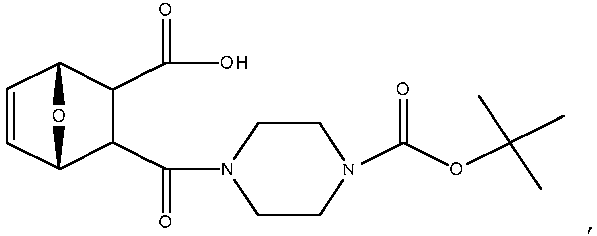 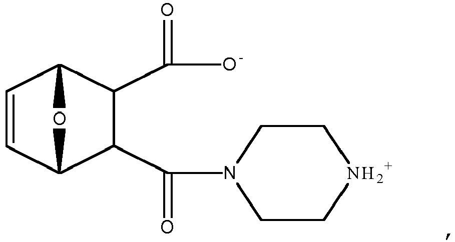 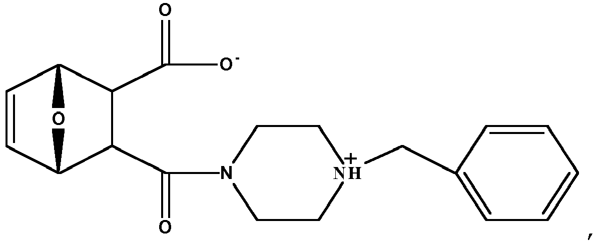 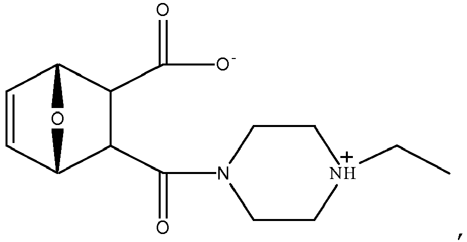 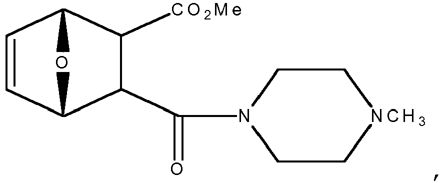 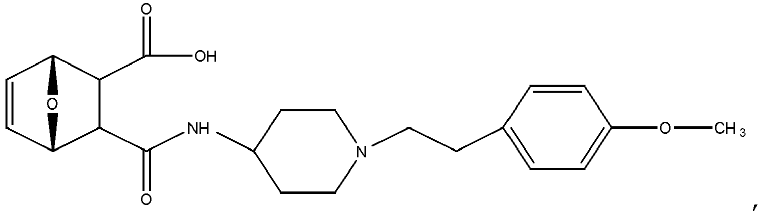 ou

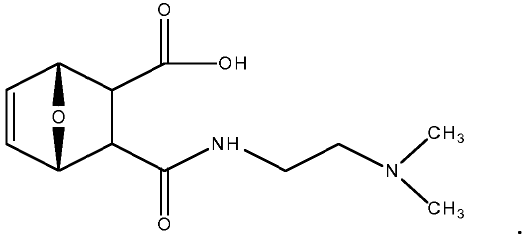

11. Inhibiteur destiné à être utilisé selon l'une quelconque des revendications 1 à 5, dans lequel l'inhibiteur de la protéine phosphatase 2A a la structure 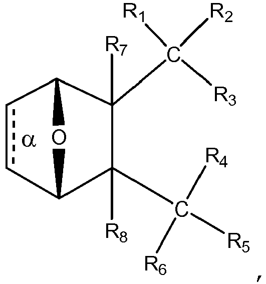 dans lequel
la liaison α est présente ou absente ;
R₁ et R₂ représentent chacun indépendamment H, O⁻ ou OR₉,
où R₉ représente H, un groupe alkyle, alcényle, alcynyle ou aryle,
ou R₁ et R₂ ensemble représentent =O ;
R₃ et R₄ sont chacun différents, et chacun représente O(CH₂)₁₋₆R₉ ou OR₉, ou 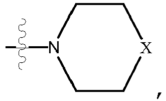 où X représente O, S, NR₁₀, ou N⁺R₁₀R₁₀,
où chaque R₁₀ représente indépendamment H, un groupe alkyle, hydroxyalkyle, alkyle en C₂ à C₁₂, alcényle, alcényle en C₄ à C₁₂, alcynyle, aryle, où le substituant est autre qu'un groupe chloro lorsque R₁ et R₂ représentent =O, 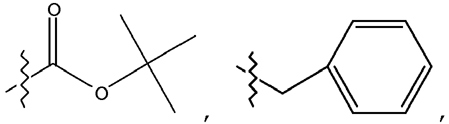 -CH₂CN, -CH₂CO₂R₁₁, -CH₂COR₁₁, -NHR₁₁ ou -NH⁺(R₁₁)₂,
où chaque R₁₁ représente indépendamment H, un groupe alkyle, alcényle ou alcynyle ;
ou R₃ et R₄ sont chacun différents et chacun représente OH ou 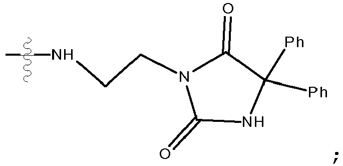 R₅ et R₆ représentent chacun indépendamment H, OH, ou R₅ et R₆ pris ensemble représentent =O ;
R₇ et R₈ représentent chacun indépendamment H, F, Cl, Br, SO₂Ph, CO₂CH₃, ou SR₁₂,
où R₁₂ représente H, un groupe aryle, un groupe substitué ou non substitué alkyle, alcényle ou alcynyle ; et
chaque occurrence des groupes alkyle, alcényle, ou alcynyle est ramifiée ou non ramifiée, non substituée ou substituée,
ou un sel, un énantiomère ou un zwittérion du composé.

12. Inhibiteur destiné à être utilisé selon la revendication 11,
dans lequel
R₁ et R₂ ensemble représentent =O ;
R₃ représente OR₉ ou O(CH₂)₁₋₂R₉,
où R₉ représente un groupe aryle, éthyle substitué, ou phényle substitué, dans lequel le substituant est en position para du groupe phényle ;
ou R₃ représente OH et R₄ représente 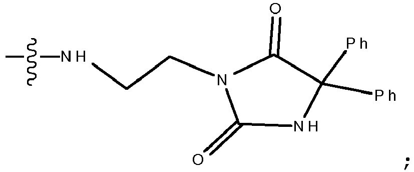 R₄ représente 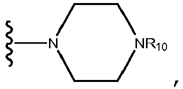 où R₁₀ représente un groupe alkyle ou hydroxyalkyle ; R₅ et R₆ ensemble représentent =O ; et
R₇ et R₈ représentent chacun indépendamment H, ou dans lequel
R₁ et R₂ ensemble représentent =O ;
R₃ représente OH, O(CH₂)R₉, ou OR₉,
où R₉ représente un groupe phényle ou CH₂CCl₃, 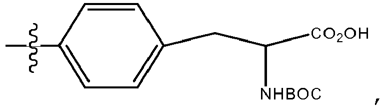 ou 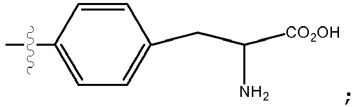 R₄ représente 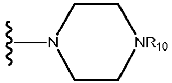 ou 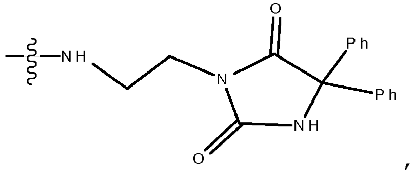 où R₁₀ représente CH₃ ou CH₃CH₂OH ;
R₅ et R₆ ensemble représentent =O ; et
R₇ et R₈ représentent chacun indépendamment H, ou dans lequel R₃ représente OR₉,
où R₉ représente (CH₂)₁₋₆(CHNHBOC)CO₂H, (CH₂)₁-₆(CHNH₂)CO₂H, (CH₂)₁₋₆CCl₃, CH₂(CHNHBOC)CO₂H, CH₂(CHNH₂)CO₂H, ou CH₂CCl₃.

13. Inhibiteur destiné à être utilisé selon la revendication 11, dans lequel l'inhibiteur de la protéine phosphatase 2A a la structure 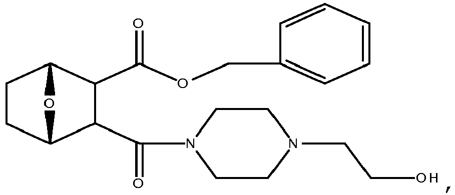 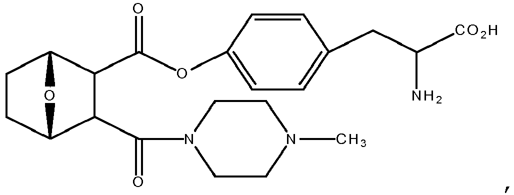 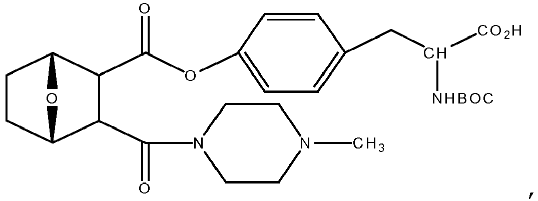 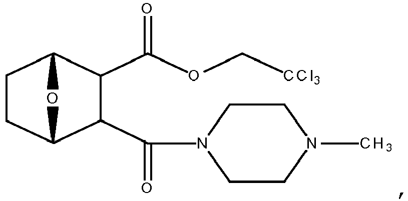 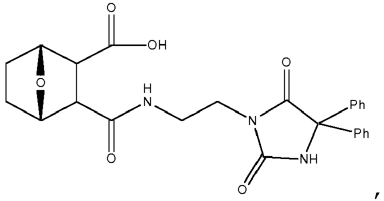 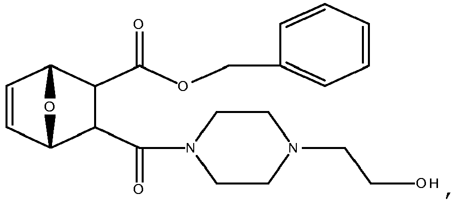 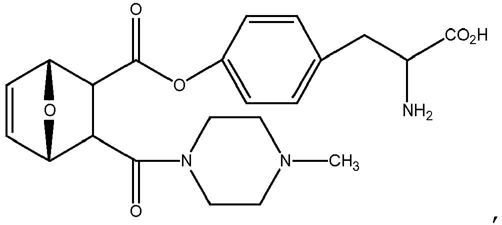 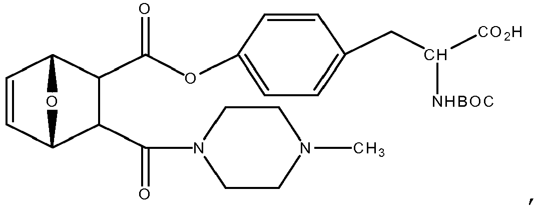 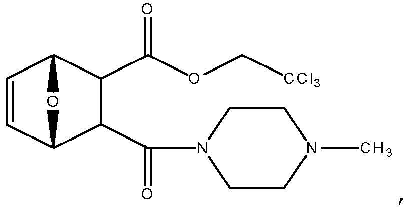 ou

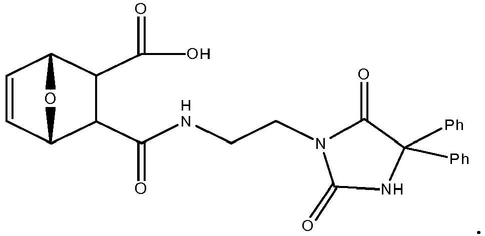

14. Inhibiteur destiné à être utilisé selon la revendication 4, dans lequel le dommage tissulaire associé à la lésion de reperfusion dans le coeur du sujet à la suite d'un infarctus du myocarde est réduit ; ou dans lequel la fuite vasculaire associée à la lésion de reperfusion chez un sujet souffrant de septicémie est réduite.

15. Inhibiteur destiné à être utilisé selon la revendication 1, dans lequel la lésion de reperfusion est le dommage tissulaire associé à la lésion de reperfusion dans le coeur du sujet à la suite d'un infarctus du myocarde et le tissu est le tissu myocardique.

16. Inhibiteur destiné à être utilisé selon la revendication 1, dans lequel la lésion de reperfusion est le dommage tissulaire dû à un traumatisme aigu chez le sujet et le tissu est le tissu endothélial.
